# EUROPÄISCHE PATENTSCHRIFT

(11) **EP 2 158 894 B1**
(45) Veröffentlichungstag und Bekanntmachung des Hinweises auf die Patenterteilung: **07.11.2018**
(21) Anmeldenummer: 09154616.8
(22) Anmeldetag: 09.03.2009
(51) Int. Cl.: A61K 8/02, A61Q 11/00, A61K 8/81, A61K 8/85, A61K 8/87

(54) **Mund- und Zahnpflege- und -reinigungsmittel**
Oral cleansing agent
Produit dentaire

(30) Priorität: 26.08.2008 DE 102008039681
(43) Veröffentlichungstag der Anmeldung: 03.03.2010
(73) Patentinhaber: Henkel AG & Co. KGaA, 40589 Düsseldorf (DE)
(72) Erfinder: Poth, Tilo, 69469, Weinheim (DE); Leinen, Hans-Theo, 40699, Erkrath (DE); Barth, Adolf Peter, 42781, Haan (DE)

(56) Entgegenhaltungen:
- EP-A1- 1 762 215
- WO-A1-2008/069631
- AU-B2- 441 530
- GB-A- 1 168 366
- GB-A- 1 309 209
- DATABASE GNPD [Online] MINTEL; August 2008 (2008-08), "Total Protection Toothpaste", XP002721396, Database accession no. 953727
- DATABASE GNPD [Online] MINTEL; März 2007 (2007-03), "Fluoride Toothpaste", XP002721397, Database accession no. 675666

## Beschreibung

Die Erfindung betrifft Mund- und Zahnpflege- und -reinigungsmittel, die eine besondere Optik aufweisen.

Zahnreinigungsmittel sind in verschiedenen Formen auf dem Markt und dienen in erster Linie der Reinigung der Zahnoberfläche und der Vorbeugung von Zahn- und Zahnfleischerkrankungen. Sie enthalten üblicherweise eine Kombination aus Poliermitteln, Feuchthaltemitteln, Tensiden, Bindemitteln, Aromastoffen und fluoridhaltigen sowie antimikrobiellen Wirkstoffen. Neben der Reinigung der Zähne erwartet der Verbraucher von den gattungsgemäßen Produkten auch eine Pflege der Zähne und der Mundhöhle. So sind insbesondere ein "sauberes" Gefühl, d.h. eine glatte und glänzende Zahnoberfläche sowie ein frisches Gefühl im Mund wesentliche Aspekte für den Kaufanreiz von Zubereitungen zur Mund- und Zahnpflege- und -reinigung. Ein erfolgreiches Mittel der gattungsgemäßen Art sollte daher die Zähne gründlich reinigen, ohne den Zahn oder die Zahnoberfläche zu schädigen und gleichzeitig Mundgeruch verringern und/oder verhindern.

Es ist bekannt, Zubereitungen zur Mund- und Zahnpflege- und -reinigung Geruchs- und/oder Geschmacksstoffe bzw. Aromen zuzusetzen, um ihnen einerseits einen besonderen Geruch bzw. Geschmack zu verleihen und andererseits um ein aromatisches Gefühl von Frische nach der Anwendung zu hinterlassen. Solche Aromen usw. sind im Bereich der Mund- und Zahnpflege und -reinigung weit verbreitet.

Es hat sich gezeigt, daß die üblicherweise eingesetzten Aromen das Verbraucherinteresse nach einem einzigartigen Produktempfinden nur unzureichend oder nur über einen bestimmten Zeitraum befriedigen. Es besteht daher nach wie vor der Bedarf, dem Verbraucher ein "typisches und unverwechselbares" Produkt zur Verfügung zu stellen, das er wiederholt kauft und anwendet.

Zur Vermeidung hygienischer Bedenken werden Zubereitungen zur Mund- und Zahnpflege- und -reinigung so verpackt, daß ein direkter Kontakt mit dem Produkt am Verkaufsort nicht möglich ist. Im Gegensatz zu anderen Kosmetika, beispielsweise Duschgelegen oder Shampoos, weisen die Verpackungen Sicherungsmittel (z.B. peel-of-Folien) auf, die verhindern, daß das Produkt am Verkaufsort Kontakt mit der Umgebung erhält. Der Verbraucher kann auch durch Abschrauben des Verschlusses das Produkt folglich nicht olfaktorisch wahrnehmen, wie dies beispielsweise bei einem Duschgel der Fall ist. Dies führt dazu, daß es weiterer Anstrengungen bedarf, das Produkt bereits am Verkaufsort als

bedarf, das Produkt bereits am Verkaufsort als "typisches und unverwechselbares" Produkt darzustellen. Hier bieten sich neben der Verpackungsgestaltung auch optische Differenzierungen des Produkts an.

Ein weiteres Aufgabenfeld besteht darin, diese optische Differenzierung einerseits möglichst "ungewöhnlich", andererseits aber möglichst haltbar bereitzustellen. Bei Streifenzahnpasten, bei denen der optische Effekt (gestreifter Strang) erst bei der Nutzung erzeugt wird, tritt dieses Problem nicht auf. Bei der Einarbeitung von Gasblasen, Partikeln usw. ist die Lagerstabilität immer ein wichtiges Problemfeld, da Sedimentation und Wandanhaftungen vermieden werden müssen, um die optische Attraktivität zu gewährleisten.

Der vorliegenden Erfindung lag die Aufgabe zugrunde, Zubereitungsformen für Mund- und Zahnpflege- und -reinigungsmittel, die bereits am Verkaufsort ein einzigartiges Empfinden hervorrufen. Dabei sollte zusätzlich eine hohe Lagerstabilität und Langzeithaltbarkeit der optischen Effekte gewährleistet werden. Es ist bekannt, Mund- und Zahnpflege- und -reinigungsmittel optisch zu differenzieren. Hier sind zunächst die verschiedenen Arten von Streifenzahnpasten zu erwähnen, die über Art, Farbe und Form sowie Lage der Streifen eine Botschaft an den Verbraucher vermitteln. Eine weitere Form der optischen Differenzierung besteht darin, farbige Partikel, Flocken Filme oder Gasblasen in die Mund- und Zahnpflege- und -reinigungsmittel zu inkorporieren.

So beschreibt die WO 2005/058265 A1 (Colgate-Palmolive) die Inkorporierung von wirkstoffbeladenen Filmen, d.h. einer Vielzahl blättchenförmiger Strukturen, die Wirkstoffe enthalten, in verschiedene Kosmetika, insbesondere in Zahnreinigungsmittel. Als Filmmaterialien werden synthetische Polymere genannt. Allerdings sind die Mund- und Zahnpflege- und -reinigungsmittel, die als Träger fungieren, hinsichtlich ihres Wassergehaltes beschränkt, da die genannten Polymere wasserlöslich oder-quellbar sind.

Es hat sich gezeigt, daß wasserlösliche oder -quellbare Polymere Probleme im Hinblick auf die Lagerstabilität hervorrufen können. Die Partikel können aneinanderhaften und entweder an der Wand kleben oder sedimentieren. Zudem verändert sich der optische Eindruck von Mitteln, welche solche Polymere enthalten, über die Zeit. Ein weiteres Problem sind wirkstoffhaltige Partikel, aus denen der Wirkstoff nach und nach in den Träger herausdiffundiert. Dies führt insbesondere bei gefärbten Partikeln zu unschönem "Ausbluten".

Die AU37772/72 offenbart eine Zahnpasta, die agglomerierte Partikel aus der gruppe der Phosphate, Carbonate, Silica, Alumina, Silikate und Methacrylat-Polymere enthält. Zahnpasten mit silberhaltigen Partikeln werden in der WO2008/069631 A1 offenbart.

Die EP 1 7 762 215 A1 offenbart Zahncremes mit stäbchenförmigen Partikeln aus Apatit, die GB-A 1 309 209 offenbart Zahncremes mit einem Gehalt an plättchenförmigen Partikeln aus Perlmutt.

Die GB-A 1 168 366 offenbart Zahnpastaformulierungen, die Partikel aus synthetischen Polymeren enthalten, welche weder plättchen- noch stäbchenförmig sind.

Der vorliegenden Erfindung lag die Aufgabe zugrunde, eine langzeitstabile optische Differenzierung unabhängig vom Wassergehalt der Mund- und Zahnpflege- und -reinigungsmittel zu ermöglichen, die auch wirkstoffbeladene Partikel ermöglicht, ohne daß es zu Ausblutungserscheinungen kommt. Überraschenderweise wurde nun festgestellt, daß die optische Differenzierung von wasserhaltigen und wasserarmen oder -freien Mund- und Zahnpflege- und -reinigungsmitteln
gelingt, indem sichtbare Partikel aus wasserunlöslichen synthetischen Materialien in diese eingearbeitet werden.

Gegenstand der vorliegenden Erfindung sind in einer ersten Ausführungsform Mund- und Zahnpflege- und -reinigungsmittel, enthaltend einen fließfähigen Träger und darin suspendierte Partikel, wobei die im fließfähigen Träger suspendierten Partikel
- mindestens anteilsweise aus wasserunlöslichen synthetischen Polymeren bestehen und
- stäbchenförmig sind, d.h. in einer Raumrichtung eine Ausdehnung aufweisen, die mindestens doppelt, vorzugsweise mindestens dreifach, besonders bevorzugt mindestens vierfach und insbesondere mindestens fünffach so groß ist wie die größere der Ausdehnungen in die beiden anderen Raumrichtungen
- in der größten räumlichen Ausdehnung Längen zwischen 100 und 10.000 µm aufweisen.

Gegenstand der vorliegenden Erfindung sind in einer zweiten Ausführungsform Mund- und Zahnpflege- und -reinigungsmittel, enthaltend einen fließfähigen Träger und darin suspendierte Partikel, wobei die im fließfähigen Träger suspendierten Partikel
- mindestens anteilsweise aus wasserunlöslichen synthetischen Polymeren bestehen und
- plättchenförmig sind, d.h. in zwei Raumrichtungen eine Ausdehnung aufweisen, die mindestens doppelt, vorzugsweise mindestens dreifach, besonders bevorzugt mindestens vierfach und insbesondere mindestens fünffach so groß ist wie die Ausdehnung in die andere Raumrichtung
- in der größten räumlichen Ausdehnung Längen zwischen 100 und 10.000 µm aufweisen.

Mund- und Zahnpflegemittel sowie Mund- und Zahnreinigungsmittel im Sinne der Erfindung sind Mund- und Zahnpasten, flüssige Mund- und Zahncremes sowie Mund- und Zahngele. Bevorzugt geeignet sind Zahnpasten und flüssige Zahnreinigungsmittel. Hierzu können die Mund- und Zahnpflege- und reinigungsmittel z.B. in Form von Zahnpasten, flüssigen Zahncremes, Mundwässern oder gegebenenfalls auch als Kaumasse, z. B. als Kaugummi, vorliegen. Bevorzugt liegen sie jedoch als mehr oder weniger fließfähige oder plastische Zahnpasten vor, wie sie zur Reinigung der Zähne unter Einsatz einer Zahnbürste verwendet werden.

Unter "fließfähig" werden im Rahmen der vorliegenden Erfindung Zusammensetzungen verstanden, die bei 20°C und 1013,25 mbar Viskositäten zwischen 0,001 und 1.000.000 mPas (=1 pPas bis 1 kPas) aufweisen. Bevorzugte erfindungsgemäße Zubereitungen weisen Viskositäten zwischen 0,1 und 500.000 mPas, vorzugsweise zwischen 1 und 250.000 mPas und insbesondere zwischen 100 und 75.000 mPas auf. In einer weiter bevorzugten Ausführungsform der Erfindung weisen die Mittel eine Viskosität [mPas] im Bereich von 8.000 bis 45.000 mPas auf (gemessen mit Brookfield RVF; Spindel 4/4UpM bei 20°C).

"Mindestens anteilsweise" kann sich im Rahmen der vorliegenden Erfindung sowohl auf die Partikelgesamtheit als auch auf den Partikel an sich beziehen. Im erstgenannten Fall enthält eine erfindungsgemäße Zusammensetzung mehrere Partikel, von denen einige aus wasserunlöslichen synthetischen Materialien bestehen, während andere Partikel nicht zwingend diesen Kriterien genügen müssen. Im letztgenannten Fall besteht der einzelne Partikel mindestens anteilsweise aus wasserunlöslichen synthetischen Materialien, d.h. er enthält diese Materialien (und ggf. weitere Materialien) oder er besteht vollständig aus ihnen.

Die erfindungsgemäßen Mund- und Zahnpflege- und -reinigungsmittel sind dadurch gekennzeichnet, daß die im fließfähigen Träger suspendierten Partikel mindestens anteilsweise aus wasserunlöslichen synthetischen Polymeren bestehen.

Der Begriff "synthetische Polymere" wird im Rahmen der vorliegenden Erfindung als Abgrenzung zum Begriff der "natürlichen" oder der "teilsynthetischen" Polymere gebraucht. Während der Begriff "natürliche Polymere" für in der Natur vorkommende Polymere, zu denen unter anderen Erfindungsgemäß bevorzugt lassen sich Polymerisate, Polykondensate und Polyaddukte als wasserunlösliche synthetische Polymere einsetzen.

Erfindungsgemäß besonders bevorzugte Mund- und Zahnpflege- und -reinigungsmittel sind dadurch gekennzeichnet, daß die im fließfähigen Träger suspendierten Partikel mindestens anteilsweise aus Polyester bestehen.

Polyester ist die Sammelbezeichnung für Polymere, deren Grundbausteine durch EsterBindungen (-CO-O-) zusammengehalten werden. Nach ihrem chemischen Aufbau lassen sich die sog. *Homopolyester* in zwei Gruppen einteilen, die Hydroxycarbonsäure-Typen (AB-Polyester) und die Dihydroxy-Dicarbonsäure-Typen (AA-BB-Polyester). Erstere werden aus nur einem einzigen Monomer durch z.B. Polykondensation einer w-Hydroxycarbonsäure oder durch Ringöffnungspolymerisation cyclischer Ester (Lactone) hergestellt. Der Aufbau letzterer erfolgt dagegen durch Polykondensation zweier komplementärer Monomerer, z.B. einem Diol und einer Dicarbonsäure. AB-Typ-Polyester sind u.a. Polyglycolsäuren (*Polyglycolide*), Polymilchsäuren (*Polylactide*), Poly(β-hydroxybuttersäure) [Poly(3-hydroxybuttersäure)], Poly(r-caprolacton)e und *Polyhydroxybenzoesäuren.*

Rein aliphatische AA-BB-Typ-Polyester sind Polykondensate aus aliphatischen Diolen und Dicarbonsäuren, die u.a. als Produkte mit endständigen Hydroxy-Gruppen (als Polydiole) für die Herstellung von Polyesterpolyurethanen eingesetzt werden [z.B. Polytetramethylenadipat]. Mengenmäßig größte technische Bedeutung haben AA-BB-Typ-Polyester aus aliphatischen Diolen und aromatischen Dicarbonsäuren, insbesondere die Polyalkylenterephthalate [Polyethylenterephthalat (PET), Polybutylenterephthalat (PBT) und Poly(1,4-cyclohexandimethylenterephthalat)e (PCDT)] als wichtigste Vertreter. Diese Typen von Polyetsern können durch Mitverwenden anderer aromatischer Dicarbonsäuren (z.B. Isophthalsäure) bzw. durch Einsatz von Diol-Gemischen bei der Polykondensation in ihren Eigenschaften breit variiert und unterschiedlichen Anwendungsgebieten angepaßt werden. Rein aromatische Polyester sind die Polyarylate, zu denen u.a. die Poly(4-hydroxybenzoesäure), Polykondensate aus Bisphenol A und Phthalsäuren oder auch solche aus Bisphenolen und Phosgen gehören.

Ein erfindungsgemäß besonders geeignetes wasserunlösliches synthetisches Polymer für die im fließfähigen Träger suspendierten Partikel ist Polyethylenterphthalat (Kurzzeichen PET, PETE). Zusammenfassend sind erfindungsgemäße Mund- und Zahnpflege- und - reinigungsmittel bevorzugt, bei denen die im fließfähigen Träger suspendierten Partikel mindestens anteilsweise aus Polyethylenterphthalat bestehen.

Ein weiteres wasserunlösliches synthetisches Polymer, das sich sehr gut als Partikelmetariel eignet, ist Polyvinylchlorid.

Polyvinylchloride (Kurzzeichen PVC) sind die bei der radikalischen Homopolymerisation von Vinylchlorid (VC) anfallende Polymeren der Struktur -[CH2-CHCl]ₙ-, bei denen die Makromoleküle nicht streng linear sind: Sie haben in Abhängigkeit vom Monomer-Umsatz und der Polymerisations-Temp. ca. 3-20 kurze Seitenketten pro 1000 C-Atome. Technische Polyvinylchloride haben Molmassen von ca. 30000-130000 g/mol, die K-Werten von 45-80 entsprechen. PVC eignet sich besonders gut, um Farbstoffe und Aromen in den Partikeln zu inkorporieren. Zusammenfassend sind erfindungsgemäße Mund- und Zahnpflege- und - reinigungsmittel bevorzugt, bei denen die im fließfähigen Träger suspendierten Partikel mindestens anteilsweise aus Polyvinylchlorid bestehen.

Weitere wasserunlösliche synthetische Polymere, die sich sehr gut als Partikelmetariel eignen, stammen aus der Gruppe der Polyurethane.

Polyurethan ist die Sammelbezeichnung für Polymere, in deren Makromolekülen die Wiederholungseinheiten durch Urethan-Gruppierungen -NH-CO-O- verknüpft sind. Technisch wichtige Polyurethane werden hergestellt aus Polyester- und/oder Polyetherdiolen und z.B. 2,4- bzw. 2,6-Toluoldiisocyanat (TDI, R² = C₆H₃-CH₃), 4,4'-Methylenbis(phenylisocyanat) (MDI, R² = C₆H₄-CH₂-C₆H₄), 4,4'-Methylendicyclohexylisocyanat (HMDI, R² = C₆H₁₀-CH₂-C₆H₁₀) od. Hexamethylendiisocyanat [HDI, R² = (CH₂)₆].

Besonders bevorzugte erfindungsgemäße Mund- und Zahnpflege- und -reinigungsmittel enthalten Partikel, die aus einem Polyurethan-Polymer bestehen, das zusammengesetzt ist aus:
a) 15 bis 35 Gew.-% an Polyisocyanat,
b) 10 bis 70, insbesondere 15 bis 35 Gew.-% an Polyalkylenglykol,
c) 5 bis 65, insbesondere 30 bis 50 Gew.-% an Polyesterglykol,
d) 0,1 bis 16, insbesondere 1 bis 8 Gew.-% an Kettenverlängerer,
e) 0 bis 15, insbesondere 3 bis 10 Gew.-% an Harz und
f) 0 bis 0,5, insbesondere 0,01 bis 0,1 Gew.-% an Stabilisator.

Wichtig ist aber nicht nur der Gewichtsbereich der einzelnen Komponenten, sondern auch ihr Gewichtsverhältnis zueinander. So sollte das Verhältnis der reaktiven Gruppen NCO : OH 1,1 : 1 bis 2 : 1 betragen, insbesondere 1,15 : 1 bis 1,5 : 1. Das für eine konkrete Polymerzusammensetzung zu wählende NCO : OH-Verhältnis ist so zu wählen, daß das Polymer ein brauchbares Molekulargewicht hat, d. h. es sollte hoch genug sein, um eine gute Anfangsfestigkeit zu ergeben, andererseits aber niedrig genug sein, damit die Viskosität - auch ohne Lösungsmittel - ausreichend niedrig sowohl bei der Herstellung als auch bei der Anwendung ist. Außerdem sollte das Polymer noch mindestens 0,5 bis 3, vorzugsweise 1,0 bis 2 g an freien NCO-Gruppen pro 100 g Polymer haben, um eine ausreichende Härtung mit Feuchtigkeit zu ergeben. Der NCO-Gehalt wird bestimmt durch Titration.

Unter einem "Polyisocyanat" ist eine niedermolekulare Verbindung mit 2 oder 3 IsocyanatGruppen zu verstehen. Die Diisocyanate werden bevorzugt, sie können aber bis zu ca. 10 Gew.-% an trifunktionellem Isocyanat enthalten. Mit steigendem Gehalt an trifunktionellem Isocyanat ist allerdings mit ungewollten Vernetzungen sowohl bei der Herstellung als auch bei der Verwendung der Polyurehan-Polymere zu rechnen. Neben aliphatischen und cycloaliphatischen Polyisocyanaten kommen vor allem aromatische Polyisocyanate in Frage. Konkrete Beispiele sind: Toluol-diisocyanat, Diphenylmethandiisocyanat und deren Mischungen. Unter Diphenylmethandiisocyanat wird sowohl das 4,4'- als auch das 2,4'-Diphenylmethandiisocyanat verstanden. Vorzugsweise sollte das 2,4'-losmere aber nicht mehr als 50 Gew.-% ausmachen. Es werden vorzugsweise ein oder zwei verschiedene Polyisocyanate eingesetzt. Vor allem wird reines 4,4'-Diphenylmethandiisocyanat verwendet. Seine Mischung mit dem 2,4'-Isomeren beeinflußt unter anderem den Gehalt an nicht umgesetztem Diisocyanat, die thermische Stabilität sowie die Länge der Reaktivierbarkeit des Befestigungselements. Der Anteil des Polyisocyanates im PU-Polymer sollte 15 bis 35, vorzugsweise 20 bis 30 Gew.-% betragen.

Unter einem "Polyalkylenglykol" wird ein linearer Polyether mit 2 OH-Gruppen verstanden. Er hat vorzugsweise die allgemeine Formel HO(-R-O)ₘ-H, wobei R ein Kohlenwasserstoffrest mit 2 bis 4 C-Atomen ist. Es kommen auch Copolymere in Frage, und zwar sowohl Blockpolymere als auch statistische Copolymere. Konkrete Polyalkylenglykole sind: Polyethylenglykol, Polytetramethylenglykol und vor allem Polypropylenglykol (R = -CH₂-CH(CH₃)-), vorzugsweise wird nur eine Art von Polyalkylenglykolen eingesetzt. Es können aber auch Mischungen verwendet werden aus 2 bis 3 Polyalkylenglykolen, die sich in ihrem mittleren Molekulargewicht oder in der Art ihrer Bausteine unterscheiden.

Die Menge des einzusetzenden Polyalkylenglykols - insbesondere des Polypropylenglykols - beträgt mindestens 10 Gew.-%, vorzugsweise 10 bis 70 und insbesondere 15 bis 35 Gew.-%, jeweils bezogen auf das Polyurehan-Polymer insgesamt.

Vor allem ist reines Polypropylenglykol interessant. Sein mittleres Molekulargewicht sollte in der Regel zwischen 250 und 1000, vorzugsweise zwischen 350 und 600 und ganz besonders zwischen 400 und 450 liegen. (Gemeint ist das Zahlenmittel aufgrund von OH-Bestimmungen).

Unter einem "Polyesterglykol" wird en Polyester mit 2 OH-Gruppen verstanden, vorzugsweise mit 2 endständigen OH-Gruppen. Sie werden auf bekanntem Wege hergestellt, sei es aus
a) aliphatischen Hydroxycarbonsäuren oder aus
b) aliphatischen Dicarbonsäuren mit 6 bis 12 C-Atomen und - insbesondere geradzähligen - Diolen mit 4 bis 8 C-Atomen.

Natürlich können auch entsprechende Derivate eingesetzt werden, z. B Lactone, Methylester oder Anhydride. Konkrete Ausgangsprodukte sind: 1,4-Butyandiol, 1,4-Hexandiol, Adipin-, Azelain-, Sebazinsäure und Lacton. Die Säurekomponente kann bis zu 25 Mol-% einer anderen Säure enthalten, z. B. Cyclohexandicarbonsäure, Terephthalsäure und Isophthalsäure. Die Glykolkomponente kann bis zu 15 Mol-% eines anderen Diols enthalten, z.B. Diethylenglykol, 1,4-Cyclohexandimethanol. Neben Homopolymeren aus obigen Bausteinen sind vor allem Copolyester aus den folgenden Bausteinen oder deren Derivaten wichtig:
1. Adipinsäure, Isophthalsäure, Phthalsäure und Butandiol,
2. Adipinsäure, Phthalsäure und Hexandiol,
3. Adipinsäure, Isophthalsäure, Phthalsäure, Ethylenglykol, Neopentylglykol und 3-Hydroxy-2,2-dimethylpropyl-3-hydroxy-2,2-dimethylpropanoat und
4. Adipinsäure, Phthalsäure, Neopentylglykol und Ethylenglykol.

Der Copolyester aus Adipinsäure, Isophthalsäure, Phthalsäure und Butandiol ist teilweise kristallin und hat eine hohe Viskosität. Er führt daher zu hohen Anfangsfestigkeiten. Der Copolyester aus Adipinsäure, Phthalsäure und Hexandiol hat eine niedrige Glasübergangstemperatur und führt daher zu einer verbesserten Kälteflexibilität.

Die Polyesterglykole sind flüssig oder fest. Wenn sie fest sind, sind sie vorzugsweise amorph. Sie können aber auch schwachkristallin sein. Vorzugsweise wird eine Mischung aus teilweise kristallinen und amorphen Polyestern eingesetzt. Die Kristallinität ist allerdings so wenig ausgeprägt, daß sie sich in dem fertigen Polyurehan-Polymer nicht durch Trübung bemerkbar macht. Der Schmelzpunkt der teilkristallinen Polyester liegt im Bereich von 40 bis 70 °C, vorzugsweise im Bereich von 45 bis 65 °C. Der Schmelzpunkt gibt die Temperatur an, bei der die kristallinen Bereiche des Materials schmelzen. Er wird differential-thermoanalytisch bestimmt durch den Endotherm-Hauptpeak. Vorzugsweise wird ein Polybutandioladipat mit einem Molekulargewicht von etwa 3 500 und einem Schmelzpunkt von etwa 50 °C als teilweise kristallines Polyesterglykol verwendet.

Das mittlere Molekulargewicht des Polyesterglykols (Mn) sollte zwischen 1 500 und 30 000 liegen, vorzugsweise zwischen 2 500 und 6 000. Es wird aus der OH-Zahl berechnet.

Unter "Kettenverlängerern" sind Verbindungen mit mehreren - insbesondere zwei - funktionellen Gruppen wie -OH, -SH, -COOH und/oder Amin zu verstehen, die ein relativ niedriges Molekulargewicht haben. Im Falle von aromatischen Kettenverlängerern liegt es unter 500, bei aliphatischen Kettenverlängerern unter 300, vorzugsweise in beiden Fällen unter 250, insbesondere unter 200. Als konkrete Verbindungen seien beispielhaft genannt:
- aromatische Kettenverlängerer wie 1,4-Bis-(ß-hydroxy-ethoxy-)benzol und ethoxyliertes und/oder propoxyliertes Bisphenol A (= 2,2-(4,4'-Dihydroxydiphenyl)-dimethylmethan,
- übliche gesättigte und ungesättigte Glykole wie Ethylenglykol oder Kondensate des Ethylenglykols, Butandiol-1,3, Butandiol-1,4, Butandiol-2,3, Propandiol-1,2, Propandiol-1,3, Neopentylglykol, Hexansiol-1,6, Bis-hydroxymethyl-cyclohexan, Dioxyethoxyhydrochinon, Terephthalsäure-bis-glykolester, Bernsteinsäure-di-2-hydroxyethylamid, Bernsteinsäure-di-N-methyl-(2-hydroxy-ethyl)amid, 1,4-Di(2-hydroxymethyl-mercapto)-2,3,5,6-tetrachlorbenzol, 2-Methylenpropandiol-(1,3),2-Methylpropandiol-(1,3), Thiodiglykol,
- aliphatische, cycloaliphatische und aromatische Diamine wie Ethylendiamin, Hexamethylendiamin, 1,4-Cyclohexylendiamin, Piperazin, N-Methyl-propylendiamin, Diaminodiphenylsulfon, Diaminodiphenylether, Diaminodiphenyldimethylmethan, 2,4-Di-amino-6-phenyltriazin, Isophorondiamin, Dimerfettsäurediamin, Diaminodiphenylmethan oder die Isomeren des Phenylendiamins. Weiterhin auch Carbohydrazide oder Hydrazide von Dicarbonsäuren,
- Aminoalkohole wie Ethanolamin, Propanolamin, Butanolamin, N-Methyl-ethanolamin, N-Methyl-isopropanolamin; Diethanolamin, Triethanolamin sowie Di- oder Tri(Alkanolamine) sowie deren Alkoxylierungsprodukte,
- aliphatische, cycloaliphatische, aromatische und heterocyclische Mono- und Diaminocarbonsäuren wie Glycin, 1- und 2-Alanin, 6-Aminocapronsäure, 4-Aminobuttersäure, die isomeren Mono- und Diaminobenzoesäure, die isomeren Mono- und Diaminonaphtheosäuren,
- Wasser.

Zur Einstellung eines geringen definierten Verzweigungsgrades können auch höherfunktionelle Kettenverlängerer in geringen Mengen eingesetzt werden, wie z. B. Trimethylolpropan oder Glycerin.

Im allgemeinen werden die Kettenverlängerer in Mengen von 0,1 bis 16, vorzugsweise 1 bis 8 Gew.-% zugegeben. Bei niedrigeren Konzentrationen nimmt der Effekt deutlich ab, bei höheren können unerwünschte Veränderungen bezüglich Haftung und Flexibilität auftreten. Natürlich können auch Mischungen von Kettenverlängerern eingesetzt werden. Zusammenfassend sind erfindungsgemäße Mund- und Zahnpflege- und -reinigungsmittel bevorzugt, bei denen die im fließfähigen Träger suspendierten Partikel mindestens anteilsweise aus Polyurethan bestehen.

Neben den genannten Homo- und/oder Copolymeren können auch Kompositmaterialien aus mehreren Polymeren, sogenannte Blends, eingesetzt werden. Hier haben sich im Rahmen der vorliegenden Erfindung insbesondere Polymerblends bewährt, die Polyethylenterephthalate enthalten. Diese lassen sich zu stabil dispergierbaren, nicht ausblutenden Partikeln formen, welche den erfindungsgemäßen Mund- und Zahnpflege- und -reinigungsmitteln eine eigenständige und langzeitstabile Optik verleihen. Insbesondere farbstoffhaltige Polymerblends, welche Polyethylenterephthalate enthalten, führen zu einer herausragenden optischen Differenzierung mit klaren, glänzenden Partikeln, ohne daß es zu Farbstoffausblutungen oder Verlust der Lagerstabilität durch Sedimentation kommt.

Ganz besonders bevorzugte erfindungsgemäße Mund- und Zahnpflege- und - reinigungsmittel enthalten im fließfähigen Träger suspendierte Partikel, die aus einem Blend aus Polyethylenterephthalat(en) und Polyacrylat(en) bestehen.

Auch Polymerblends aus PET und PU sind erfindungsgemäß vorteilhaft einsetzbar. Auch hier sind Lagerstabilität und Verhinderung der Ausblutungsneigung hervorragend. Weiter bevorzugte erfindungsgemäße Mund- und Zahnpflege- und -reinigungsmittel sind demnach dadurch gekennzeichnet, daß die im fließfähigen Träger suspendierten Partikel aus einem Blend aus Polyethylenterephthalat(en) und Polyurethan(en) bestehen.

Besonders bevorzugte Blends dieser Ausführungsform enthalten 90 bis 99 Gew.-% PET und 1 bis 10 Gew.-% PU, jeweils bezogen auf die Polymerpartikel. Ganz besonders bevorzugte Blends enthalten 95 bis 98,5 Gew.-% PET und 1,5 bis 5 Gew.-% PU, noch weitere bevorzugt sind Blends mit 97 bis 98 Gew.-% PET und 2 bis 3 Gew.-% PU.

Anstelle von wasserunlöslichen synthetischen Polymeren oder zusätzlich zu ihnen können die erfindungsgemäßen Mund- und Zahnpflege- und -reinigungsmittel auch im fließfähigen Träger suspendierte Partikel enthalten, die mindestens anteilsweise aus wasserunlöslichen synthetischen Materialien aus der Gruppe der Metalle bestehen.

Ganz besonders bevorzugte erfindungsgemäße Mund- und Zahnpflege- und -reinigungsmittel enthalten im fließfähigen Träger suspendierte Partikel, die aus einem Blend aus Polyethylenterephthalat(en) und Polyacrylat(en) bestehen.

Auch Polymerblends aus PET und PU sind erfindungsgemäß vorteilhaft einsetzbar. Auch hier sind Lagerstabilität und Verhinderung der Ausblutungsneigung hervorragend. Weiter bevorzugte erfindungsgemäße Mund- und Zahnpflege- und -reinigungsmittel sind demnach dadurch gekennzeichnet, daß die im fließfähigen Träger suspendierten Partikel aus einem Blend aus Polyethylenterephthalat(en) und Polyurethan(en) bestehen.

Besonders bevorzugte Blends dieser Ausführungsform enthalten 90 bis 99 Gew.-% PET und 1 bis 10 Gew.-% PU, jeweils bezogen auf die Polymerpartikel. Ganz besonders bevorzugte Blends enthalten 95 bis 98,5 Gew.-% PET und 1,5 bis 5 Gew.-% PU, noch weitere bevorzugt sind Blends mit 97 bis 98 Gew.-% PET und 2 bis 3 Gew.-% PU.

Besonders reizvolle optische Effekte lassen sich durch Kompositmaterialien aus Metall(en) und Kunststoff(en) realisieren. Diese Kompositmaterialien zeichnen sich darüber hinaus durch eine hervorragende Sedimentationsstabilität, äußert geringe Wandanhaftung und eine praktisch nicht vorhandene Ausblutungsneigung aus. Ganz besonders bevorzugte erfindungsgemäße Mund- und Zahnpflege- und -reinigungsmittel sind daher dadurch gekennzeichnet, daß die im fließfähigen Träger suspendierten Partikel mindestens anteilsweise aus Kompositmaterialien aus synthetischen Polymeren und Metall(en) bestehen.

Solche Kompositmaterialien sind kommerziell erhältlich und werden beispielsweise von der Glitterex Corporation, Cranford NJ, USA unter den Bezeichnungen "Disco*Flake", "Disco*ET", "Disco*HT", "Disco-LR", "Holo*Gram", "Poly*Flake", "PVC" oder "Ultra*Brite" vertrieben. Es handelt sich dabei um metallisierte PVC-Folien oder Polyesterfolien.

Die Produkte sind in einer breiten Farb- und Formenvielfalt erhältlich, beispielsweise als Quadrate mit Kantenlängen von 100 µm, 200 µm, 375 µm, 625 µm, 875 µm, 1550 µm, 3125 µm und 4675 µm, regelmäßige Sechsecke mit Seitenlängen von 100 µm, 150 µm, 200 µm, 275 mm, 375 µm, 625 µm, 875 µm, 1550 µm, 3125 µm, 4675 µm und 6250 µm, als Rechtecke in den Abmessungen 50 µm x 75 µm, 100 µm x 200 µm, 200 µm x 375 µm, 375 µm x 625 µm als Fäden

Härtegrade zwischen 0 und 20 °dH auf. Besonders bevorzugt ist der Einsatz von technisch vollentsalztem Wasser, das beispielsweise durch lonenaustausch gewonnen werden kann. Weitere Inhaltsstoffe des fließfähigen Trägers werden weiter unten ausführlich beschrieben. Vorzugsweise sind die Partikel mit Wirk- und/oder Hilfsstoffen beladen. Hier bieten sich beispielsweise Farbstoffe, Duftstoffe, Öle, Aromen, Antikarieswirkstoffe, Wirkstoffe gegen Halitosis usw. an. Besonders bevorzugte erfindungsgemäße Mund- und Zahnpflege- und - reinigungsmittel sind dadurch gekennzeichnet, daß die im fließfähigen Träger suspendierten Partikel - bezogen auf ihr Gewicht - 0,01 bis 20 Gew.-% Duftstoff(e) und/oder Aromen enthalten.

Als wasserunlösliche Aromaöle kommen alle für Mund- und Zahnpflegemittel gebräuchlichen natürlichen und synthetischen Aromen infrage. Natürliche Aromen können sowohl in Form der aus den Drogen isolierten etherischen Öle als auch in Form der aus diesen isolierten Einzelkomponenten verwendet werden. Bevorzugt sollte wenigstens ein Aromaöl aus der Gruppe Pfefferminzöl, Krauseminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte oder synthetisch erzeugte Komponenten dieser Öle enthalten sein. Die wichtigsten Komponenten der genannten Öle sind z. B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z. B. Menthylacetat, Vanillin, Jonone, Linalylacetat, Rhodinol und Piperiton.

Besonders bevorzugte erfindungsgemäße Mund- und Zahnpflege- und -reinigungsmittel sind dadurch gekennzeichnet, daß die im fließfähigen Träger suspendierten Partikel - bezogen auf ihr Gewicht - 0,05 bis 19 Gew.-%, vorzugsweise 0,1 bis 18 Gew.-%, besonders bevorzugt 0,5 bis 17 Gew.-%, weiter bevorzugt 0,75 bis 16 Gew.-% und insbesondere 1 bis 15 Gew.-% mindestens eines Aromas aus der Gruppe Pfefferminz, Spearmint, Menthol, Limone, Limette, Apfel, Orange, Zitrone, Erdbeere, Aloe Vera, Kaugummi enthalten.

Die im fließfähigen Träger suspendierten Partikel können zusätzlich zu Aromen und/oder Farbstoffen oder an deren Stelle auch scharf schmeckende oder ein Gefühl von Wärme erzeugende Substanzen enthalten. Als scharf schmeckende oder ein Gefühl von Wärme erzeugende Substanz kann eine Reihe von Stoffen eingesetzt werden. Bevorzugt sind insbesondere N-Alkyl-substituierte Amide von ungesättigten Carbonsäuren, beispielsweise 2E,4E-Decadiensäure-N-Methylamid, 2E,4E-Decadiensäure-N-Ethylamid, 2E,4E-Decadiensäure-N-n-Propylamid, 2E,4E-Decadiensäure-N-Isopropylamid, 2E,4E-Decadiensäure-N-n-Butylamid, 2E,4E-Decadiensäure-N-(1-Methylpropyl)-amid, 2E,4E-Decadiensäure-N-Isobutylamid, 2E,4E-Decadiensäure-N-tert-Butylamid, 2E,4Z-Decadiensäure-N-Methylamid, 2E,4Z-Decadiensäure-N-Ethylamid, 2E,4Z-Decadiensäure-N-n-Propylamid, 2E,4Z-Decadiensäure-N-Isopropylamid, 2E,4Z-Decadiensäure-N-n-Butylamid, 2E,4Z-Decadiensäure-N-(1-Methylpropyl)-amid, 2E,4Z-Decadiensäure-N-Isobutylamid, 2E,4Z-Decadiensäure-N-tert-Butylamid, 2E,4E,8Z-Decatriensäure-N-Methylamid, 2E,4E,8Z-Decatriensäure-N-Ethylamid, 2E,4E,8Z-Decatriensäure-N-n-Propylamid, 2E,4E,8Z-Decatriensäure-N-Isopropylamid, 2E,4E,8Z-Decatriensäure-N-n-Butylamid, 2E,4E,8Z-Decatriensäure-N-(1-Methylpropyl)-amid, 2E,4E,8Z-Decatriensäure-N-Isobutylamid, 2E,4E,8Z-Decatriensäure-N-tert-Butylamid, 2E,4Z,8Z-Decatriensäure-N-Methylamid, 2E,4Z,8Z-Decatriensäure-N-Ethylamid, 2E,4Z,8Z-Decatriensäure-N-n-Propylamid, 2E,4Z,8Z-Decatriensäure-N-Isopropylamid, 2E,4Z,8Z-Decatriensäure-N-n-Butylamid, 2E,4Z,8Z-Decatriensäure-N-(1-Methylpropyl)-amid, 2E,4Z,8Z-Decatriensäure-N-Isobutylamid, 2E,4Z,8Z-Decatriensäure-N-tert-Butylamid, 2E,4E,8E-Decatriensäure-N-Methylamid, 2E,4E,8E-Decatriensäure-N-Ethylamid, 2E,4E,8E-Decatriensäure-N-n-Propylamid, 2E,4E,8E-Decatriensäure-N-Isopropylamid, 2E,4E,8E-Decatriensäure-N-n-Butylamid, 2E,4E,8E-Decatriensäure-N-(1-Methylpropyl)-amid, 2E,4E,8E-Decatriensäure-N-Isobutylamid, 2E,4E,8E-Decatriensäure-N-tert-Butylamid, 2E,4Z,8E-Decatriensäure-N-Methylamid, 2E,4Z,8E-Decatriensäure-N-Ethylamid, 2E,4Z,8E-Decatriensäure-N-n-Propylamid, 2E,4Z,8E-Decatriensäure-N-Isopropylamid, 2E,4Z,8E-Decatriensäure-N-n-Butylamid, 2E,4Z,8E-Decatriensäure-N-(1-Methylpropyl)-amid, 2E,4Z,8E-Decatriensäure-N-Isobutylamid, 2E,4Z,8E-Decatriensäure-N-tert-Butylamid, 2E,6Z,8E-Decatriensäure-N-Methylamid, 2E,6Z,8E-Decatriensäure-N-Ethylamid, 2E,6Z,8E-Decatriensäure-N-n-Propylamid, 2E,6Z,8E-Decatriensäure-N-Isopropylamid, 2E,6Z,8E-Decatriensäure-N-n-Butylamid, 2E,6Z,8E-Decatriensäure-N-(1-Methylpropyl)-amid, 2E,6Z,8E-Decatriensäure-N-Isobutylamid, 2E,6Z,8E-Decatriensäure-N-tert-Butylamid, 2E,6E,8E-Decatriensäure-N-Methylamid, 2E,6E,8E-Decatriensäure-N-Ethylamid, 2E,6E,8E-Decatriensäure-N-n-Propylamid, 2E,6E,8E-Decatriensäure-N-Isopropylamid, 2E,6E,8E-Decatriensäure-N-n-Butylamid, 2E,6E,8E-Decatriensäure-N-(1-Methylpropyl)-amid, 2E,6E,8E-Decatriensäure-N-Isobutylamid, 2E,6E,8E-Decatriensäure-N-tert-Butylamid, 2E,6Z,8Z-Decatriensäure-N-Methylamid, 2E,6Z,8Z-Decatriensäure-N-Ethylamid, 2E,6Z,8Z-Decatriensäure-N-n-Propylamid, 2E,6Z,8Z-Decatriensäure-N-Isopropylamid, 2E,6Z,8Z-Decatriensäure-N-n-Butylamid, 2E,6Z,8Z-Decatriensäure-N-(1-Methylpropyl)-amid, 2E,6Z,8Z-Decatriensäure-N-Isobutylamid, 2E,6Z,8Z-Decatriensäure-N-tert-Butylamid, 2E,6E,8Z-Decatriensäure-N-Methylamid, 2E,6E,8Z-Decatriensäure-N-Ethylamid, 2E,6E,8Z-Decatriensäure-N-n-Propylamid, 2E,6E,8Z-Decatriensäure-N-Isopropylamid, 2E,6E,8Z-Decatriensäure-N-n-Butylamid, 2E,6E,8Z-Decatriensäure-N-(1-Methylpropyl)-amid, 2E,6E,8Z-Decatriensäure-N-Isobutylamid, 2E,6E,8Z-Decatriensäure-N-tert-Butylamid, 2E,7Z,9E-Undecatriensäure -N-Methylamid, 2E,7Z,9E-Undecatriensäure -N-Ethylamid, 2E,7Z,9E-Undecatriensäure -N-n-Propylamid, 2E,7Z,9E-Undecatriensäure -N-Isopropylamid, 2E,7Z,9E-Undecatriensäure -N-n-Butylamid, 2E,7Z,9E-Undecatriensäure -N-(1-Methylpropyl)-amid, 2E,7Z,9E-Undecatriensäure-N-isobutylamid, 2E,7Z,9E-Undecatriensäure -N-tert-Butylamid, 2E,7E,9E-Undecatriensäure -N-Methylamid, 2E,7E,9E-Undecatriensäure -N-Ethylamid, 2E,7E,9E-Undecatriensäure -N-n-Propylamid, 2E,7E,9E-Undecatriensäure -N-Isopropylamid, 2E,7E,9E-Undecatriensäure -N-n-Butylamid, 2E,7E,9E-Undecatriensäure -N-(1-Methylpropyl)-amid, 2E,7E,9E-Undecatriensäure-N-isobutylamid, 2E,7E,9E-Undecatriensäure -N-tert-Butylamid, 2E,7Z,9Z-Undecatriensäure -N-Methylamid, 2E,7Z,9Z-Undecatriensäure -N-Ethylamid, 2E,7Z,9Z-Undecatriensäure -N-n-Propylamid, 2E,7Z,9Z-Undecatriensäure -N-Isopropylamid, 2E,7Z,9Z-Undecatriensäure -N-n-Butylamid, 2E,7Z,9Z-Undecatriensäure -N-(1-Methylpropyl)-amid, 2E,7Z,9Z-Undecatriensäure-N-isobutylamid, 2E,7Z,9Z-Undecatriensäure -N-tert-Butylamid, 2E,7Z,9E-Undecatriensäure -N-Methylamid, 2E,7Z,9E-Undecatriensäure -N-Ethylamid, 2E,7Z,9E-Undecatriensäure -N-n-Propylamid, 2E,7Z,9E-Undecatriensäure -N-Isopropylamid, 2E,7Z,9E-Undecatriensäure -N-n-Butylamid, 2E,7Z,9E-Undecatriensäure -N-(1-Methylpropyl)-amid, 2E,7Z,9E-Undecatriensäure-N-isobutylamid, 2E,7Z,9E-Undecatriensäure -N-tert-Butylamid.

Selbstverständlich sind auch andere Substitutionsmuster am Stickstoffatom möglich und bevorzugt, beispielsweise längerkettige n-Alkylreste (...-N-n-Pentylamid, ...-N-n-Pentylamid, ...-N-n-Pentylamid, ...-N-n-Pentylamid, ...-N-n-Pentylamid, ...-N-n-Hexylamid, ...-N-n-Heptylamid, ...-N-n-Octylamid, ...-N-n-Nonylamid, ...-N-n-Decylamid, ...-N-n-Undecylamid, ...-N-n-Dodecylamid, ...-N-n-Tridecylamid, usw.) oder disubstituierte ...-N,N-Dialkylamide wie ...-N,N-dimethylamid, ...-N,N-diethylamid, ...-N,N-di-n-propylamid, ...-N,N-diisopropylamid, ...-N,N-di-n-butylamid, ...-N,N-di(1-Methylpropyl)amid, ...-N,N-diisobutylamid, ...-N,N-di-tert-butylamid, ...-N,N-methyl-ethylamid, ...-N,N-methyl-n-propylamid, ...-N,N-methyl-isopropylamid, ...-N,N-ethyl-n-propylamid, ...-N,N-ethyl-isopropylamid, usw..

Unter den genannten Verbindungen sind einige im Rahmen der vorliegenden Erfindung besonders bevorzugt. Diese sind nachfolgend aufgeführt:
2E,6Z,8E-Decatriensäure-N-isobutylamid (N-Isobutyl-2E,6Z,8E-decatrienamid, auch Spilanthol oder Affinin genannt).
2E,4E,8Z-Decatriensäure-N-isobutylamid (N-Isobutyl-2E,4E,8Z-decatrienamid, auch Isoaffinin genannt).
2E,7Z,9E-Undecatriensäure-N-isobutylamid (N-Isobutyl-2E,7Z,9E-undecatrienamid). 2E,4Z-Decadiensäure-N-isobutylamid (cis-Pellitorin).
2E,4E-Decadiensäure-N-isobutylamid (trans-Pellitorin). Ferulasäureamide, beispielsweise Ferulasäure-N-Vanillylamid.
*N*-[2-(4-Hydroxy-3-methoxyphenyl)ethyl]-3-(4-hydroxy-3-methoxy-phenyl)- (2*E*)-propensäureamid (trans-Feruloylmethoxytyramin).
*N*-[2-(4-Hydroxy-3-methoxyphenyl)ethyl]-3-(4-hydroxy-3-methoxy-phenyl)-(2*Z*)-propensäureamid (cis-Feruloylmethoxytyramin).
*N*-[2-(4-Hydroxy-3-methoxyphenyl)ethyl]-3-(4-hydroxy-3-methoxy-phenyl)- propansäureamid (Dihydroferuloylmethoxytyramin).
*N*-[2-(3,4-Dihydroxyphenyl)ethyl]-3-(4-hydroxy-3-methoxy-phenyl)-(2*E*)- propensäureamid (trans-Feruloyldopamin).
*N*-[2-(3,4-Dihydroxyphenyl)ethyl]-3-(4-hydroxy-3-methoxy-phenyl)-(2*Z*)-propensäureamid (cis- Feruloyldopamin).
*N*-[2-(4-Hydroxyphenyl)ethyl]-3-(3,4-dihydroxyphenyl)-(2*E*)-propensäureamid (trans-Caffeoyltyramin).
*N*-[2-(4-Hydroxyphenyl)ethyl]-3-(3,4-dihydroxyphenyl)-(2Z)-propensäureamid (cis-Caffeoyltyramin).
*N*-[2-(3,4-Dimethoxyphenyl)ethyl]-3-(3,4-dimethoxyphenyl)-(2*E*)-propensäureamid (trans-Rubenamin).
*N*-[2-(3,4-dimethoxyphenyl)ethyl]-3-(3,4-dimethoxy-phenyl)-(2*Z*)-propensäureamid (cis-Rubenamin).

Weitere im Rahmen der vorliegenden Erfindung mit besonderem Vorzug einsetzbare Scharfstoffe sind beispielsweise Extrakte aus Naturpflanzen. Scharf schmeckende pflanzliche Extrakte können alle physiologisch unbedenklichen pflanzlichen Extrakte sein, die einen scharfen oder warmen sensorischen Eindruck hervorrufen. Bevorzugt als scharf schmeckende pflanzliche Extrakte sind beispielsweise Pfefferextrakt (*Piper ssp.,* insbesondere *Piper nigrum*), Wasserpfefferextrakt (*Polygonum ssp.* ,insbesondere *Polygonum hydropiper*), Extrakte aus *Allium* ssp.(insbesondere Zwiebel und Knoblauchextrakte), Extrakte aus Rettich (*Raphanus ssp*.), Meerrettichextrakte (*Cochlearia armoracia*)*,* Extrakte aus schwarzem (*Brassica nigra*), wildem oder gelbem Senf (*Sinapis* ssp., insbesondere *Sinapis arvensis* und *Sinapis alba*), Bertramwurzel-Extrakte (*Anacyclus* ssp., insbesondere *Anacyclus pyrethrumL*.), Sonnenhutextrakte (*Echinaceae ssp*.), Extrakten aus Szechuan-Pfeffer (*Zanthoxylum ssp*., insbesondere *Zanthoxylum piperitum*)*,* Spilanthesextrakt (*Spilanthes ssp.,* insbesondere *Spilanthes acmella*), Chiliextrakt (*Capsicum* ssp., insbesondere *Capsicum frutescens*), Paradieskörner-Extrakt (*Aframomum ssp*.,insbesondere *Aframomum* melegueta[Rose] K. Schum.),Ingwerextrakt (*Zingiber ssp*.,insbesondere *Zingiber officinale*) und Galangaextrakt (*Kaempferia galanga* oder *Alpinia galanga*).

Eine besonders geeignete Substanz ist das aus dem Ingwerextrakt stammende Gingerol:

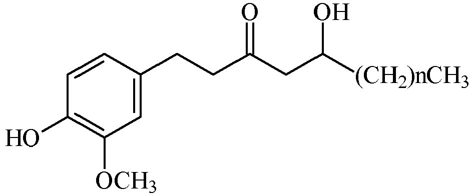

Einsetzbar ist auch N-Ethyl-p-menthan-3-carboxamid (N-Ethyl-5-Methyl-2-isopropylcyclohexancarboxamid).

Andere scharf schmeckende oder ein Gefühl von Wärme erzeugende Substanzen können z.B. sein Capsaicin, Dihydrocapsaicin, Gingerol, Paradol, Shogaol, Piperin, Carbonsäure-N-vanillylamide, insbesondere Nonansäure-N- vanillylamid, 2-Alkensäureamide, insbesondere 2-Nonensäure-N-isobutylamid, 2- Nonensäure-N-4-hydroxy-3- methoxyphenylamid, Alkylether von 4-Hydroxy-3- methoxybenzylalkohol, insbesonders 4-Hydroxy-3-methoxybenzyl-n-butylether, Alkylether von 3- Hydroxy-4-methoxybenzylalkohol, Alkylether von 3,4- Dimethoxybenzylalkohol, Alkylether von 3-Ethoxy-4-hydroxybenzylalkohol, Alkylether von 3,4-Methylendioxybenzylalkohol, Nicotinaldehyd, Methylnicotinat, Propylnicotinat, 2-Butoxyethylnicotinat, Benzylnicotinat, 1-Acetoxychavicol, Polygodial oder Isodrimeninol. Besonders bevorzugte erfindungsgemäße Mund- und Zahnpflege- und -reinigungsmittel sind dadurch gekennzeichnet, daß die im fließfähigen Träger suspendierten Partikel mindestens einen Scharfstoff aus der Gruppe der N-Alkyl-substituierte Amide von ungesättigten Carbonsäuren, vorzugsweise
- 2E,6Z,8E-Decatriensäure-N-isobutylamid (Spilanthol) und/oder
- 2E,4E,8Z-Decatriensäure-N-isobutylamid und/oder
- 2E,7Z,9E-Undecatriensäure-N-isobutylamid und/oder
- 2E,4Z-Decadiensäure-N-isobutylamid (cis-Pellitorin) und/oder
- 2E,4E-Decadiensäure-N-isobutylamid (trans-Pellitorin) und/oder
- Ferulasäure-N-Vanillylamid und/oder
- *N*-[2-(4-Hydroxy-3-methoxyphenyl)ethyl]-3-(4-hydroxy-3-methoxy-phenyl)-(2*E*)-propensäureamid (trans-Feruloylmethoxytyramin) und/oder
- *N-[2-* (4-Hydroxy-3-methoxyphenyl)ethyl]-3-(4-hydroxy-3-methoxy-phenyl)-(2 Z)-propensäureamid (cis-Feruloylmethoxytyramin) und/oder
- *N*-[2-(4-Hydroxy-3-methoxyphenyl)ethyl]-3-(4-hydroxy-3-methoxy-phenyl)-propansäureamid (Dihydroferuloylmethoxytyramin) und/oder
- *N*-[2-(3,4-Dihydroxyphenyl)ethyl]-3-(4-hydroxy-3-methoxy-phenyl)-(2*E*)-propensäureamid (trans-Feruloyldopamin) und/oder
- *N*-[2-(3,4-Dihydroxyphenyl) ethyl]-3-(4-hydroxy-3-methoxy-phenyl)-(2*Z*)-propensäureamid (cis- Feruloyldopamin) und/oder
- *N*-[2-(4-Hydroxyphenyl)ethyl]-3-(3,4-dihydroxyphenyl)-(2*E*)-propensäureamid (trans-Caffeoyltyramin) und/oder
- *N*-[2-(4-Hydroxyphenyl)ethyl]-3-(3,4-dihydroxyphenyl)-(2*Z*) propensäureamid (cis-Caffeoyltyramin) und/oder
- *N*-[2-(3,4-Dimethoxyphenyl)ethyl]-3-(3,4-dimethoxyphenyl)-(2*E*)-propensäureamid (trans-Rubenamin) und/oder
- *N*-[2-(3,4-dimethoxyphenyl)ethyl]-3-(3,4-dimethoxy-phenyl)-(2*Z*)-propensäureamid (cis-Rubenamin)
enthalten

Bei der Herstellung der suspendierten Partikel kann die Einarbeitung von Wirkstoffen durch den Einsatz von Tensiden erleichtert werden. Diese Tenside verbleiben naturgemäß im Partikel. Zudem wird die Partikelsuspension durch den Tensideinsatz stabilisiert, so daß bevorzugte erfindungsgemäße Mund- und Zahnpflege- und -reinigungsmittel dadurch gekennzeichnet sind, daß die im fließfähigen Träger suspendierten Partikel - bezogen auf ihr Gewicht - zusätzlich 0,01 bis 5 Gew.-%, vorzugsweise 0,05 bis 2,5 Gew.-% und insbesondere 0,1 bis 1 Gew.-% Tensid(e) enthalten, wobei bevorzugte Tenside ausgewählt sind aus Fettalkoholsulfaten, insbesondere Natriumlaurylsulfat, und/oder Betainen, insbesondere Cocoamidopropylbetain.
- Ferulasäure-N-Vanillylamid und/oder
- *N*-[2-(4-Hydroxy-3-methoxyphenyl)ethyl]-3-(4-hydroxy-3-methoxy-phenyl)- (2*E*)-propensäureamid (trans-Feruloylmethoxytyramin) und/oder
- *N*-[2-(4-Hydroxy-3-methoxyphenyl)ethyl]-3-(4-hydroxy-3-methoxy-phenyl)-(2*Z*)-propensäureamid (cis-Feruloylmethoxytyramin) und/oder
- *N*-[2-(4-Hydroxy-3-methoxyphenyl)ethyl]-3-(4-hydroxy-3-methoxy-phenyl)-propansäureamid (Dihydroferuloylmethoxytyramin) und/oder
- *N*-[2-(3,4-Dihydroxyphenyl)ethyl]-3-(4-hydroxy-3-methoxy-phenyl)-(2*E*)-propensäureamid (trans-Feruloyldopamin) und/oder
- *N*-[2-(3,4-Dihydroxyphenyl) ethyl]-3-(4-hydroxy-3-methoxy-phenyl)-(2Z)-propensäureamid (cis-Feruloyldopamin) und/oder
- *N*-[2-(4-Hydroxyphenyl)ethyl]-3-(3,4-dihydroxyphenyl)-(2*E*)-propensäureamid (trans-Caffeoyltyramin) und/oder
- *N*-[2-(4-Hydroxyphenyl)ethyl]-3-(3,4-dihydroxyphenyl)-(2*Z*)-propensäureamid (cis-Caffeoyltyramin) und/oder
- *N*-[2-(3,4-Dimethoxyphenyl) ethyl]-3-(3,4-dimethoxyphenyl)-(2*E*)-propensäureamid (trans-Rubenamin) und/oder
- *N*-[2-(3,4-dimethoxyphenyl)ethyl]-3-(3,4-dimethoxy-phenyl)-(2*Z*)-propensäureamid (cis-Rubenamin)
enthalten

Bei der Herstellung der suspendierten Partikel kann die Einarbeitung von Wirkstoffen durch den Einsatz von Tensiden erleichtert werden. Diese Tenside verbleiben naturgemäß im Partikel. Zudem wird die Partikelsuspension durch den Tensideinsatz stabilisiert, so daß bevorzugte erfindungsgemäße Mund- und Zahnpflege- und -reinigungsmittel dadurch gekennzeichnet sind, daß die im fließfähigen Träger suspendierten Partikel - bezogen auf ihr Gewicht - zusätzlich 0,01 bis 5 Gew.-%, vorzugsweise 0,05 bis 2,5 Gew.-% und insbesondere 0,1 bis 1 Gew.-% Tensid(e) enthalten, wobei bevorzugte Tenside ausgewählt sind aus Fettalkoholsulfaten, insbesondere Natriumlaurylsulfat, und/oder Betainen, insbesondere Cocoamidopropylbetain.

Bevorzugte Formen der ersten Ausführungsform der vorliegenden Erfindung (stäbchenförmige Partikel) sind beispielsweise Fäden oder lange Zylinder.

Eine weitere bevorzugte geometrische Form, die die suspendierten Partikel einnehmen können, ist plättchenförmig, siehe die zweite Ausführungsform der vorliegenden Erfindung. In dieser Ausführungsform können flächige Gestaltungen realisiert werden. Neben der Form der "Gehwegplatte" oder "Wandfliese" (Quadrat bzw. Rechteck mit einer Höhe, die deutlich kleiner ist als die Seitenlängen) können Kreise, Dreiecke, Fünfecke, Sechsecke, Pluszeichen, Ringe usw. gewählt werden. Auch irreguläre Formen wie Bäume, Sterne, Tierformen, Wolken, Buchstaben, Zahlen usw. sind bevorzugt. Es ist möglich und bevorzugt, daß nicht nur Partikel einer Gestaltung in den erfindungsgemäßen Produkten vorliegen, sondern mehrere Partikelserien mit einer Vielzahl von Partikeln mit jeweils gleicher Gestaltung, beispielsweise Kreise und Ringe.

Auch hinsichtlich der Größe der in den erfindungsgemäßen Mitteln enthaltenen Partikel ist die Erfindung keinen Beschränkungen unterworfen. Bevorzugt sind Mund- und Zahnpflege- und -reinigungsmittel, bei denen die im fließfähigen Träger suspendierten Partikel in der größten räumlichen Ausdehnung Längen zwischen 200 und 8000 µm, besonders bevorzugt zwischen 500 und 7000 µm und insbesondere zwischen 1000 und 5000 µm aufweisen.

Die erfindungsgemäßen Mund- und Zahnpflege- und -reinigungsmittel enthalten üblicherweise weitere Inhaltsstoffe.

Bevorzugt ist hierbei der Einsatz von sogenannten Feuchthaltemitteln, die bei Zahnpasten das Austrocknen verhindern. Bei sogenannten Flüssigzahncremes mit fließfähiger Rheologie dienen diese als Matrix und werden in höheren Mengen eingesetzt.

Hier sind erfindungsgemäße Mund und Zahnpflege- und -reinigungsmittel bevorzugt, die - bezogen auf ihr Gewicht - 10 bis 60 Gew.-%, vorzugsweise 12,5 bis 50 Gew.-%, besonders bevorzugt 15 bis 45 Gew.-% und insbesondere 20 bis 40 Gew.-% mindestens eines mehrwertigen Alkohols aus der Gruppe Sorbit und/oder Glycerin und/oder 1,2-Propylenglycol.-% enthalten.

Für bestimmte Anwendungsbereiche kann es vorteilhaft sein, nur einen der drei oben genannten Inhaltsstoffe einzusetzen. In den meisten Fällen ist dabei Sorbit bevorzugt. Allerdings können auf anderen Anwendungsgebieten Mischungen von zwei der drei Stoffe oder aller drei Stoffe bevorzugt sein. Besonders vorteilhaft hat sich hier eine Mischung aus Glycerin, Sorbit und 1,2-Propylenglycol in einem Gewichtsverhältnis von 1 : (0,5-1): (0,1-0,5) erwiesen.

Besonders bevorzugt sind Zahnpasten und flüssige Zahnreinigungsmittel, die als Poliermittel Kieselsäuren enthalten. Geeignete Kieselsäuren sind z.B. Gelkieselsäuren, Hydrogelkieselsäuren und Fällungskieselsäuren. Gelkieselsäuren werden durch Umsetzung von Natriumsilikatlösungen mit starken, wäßrigen Mineralsäuren unter Ausbildung eines Hydrosols, Alterung zum Hydrogel, Waschen und Trocknen hergestellt. Erfolgt die Trocknung unter schonenden Bedingungen auf Wassergehalte von 15 bis 35 Gew.-%, so werden die sogenannten Hydrogelkieselsäuren erhalten. Durch Trocknung auf Wassergehalte unterhalb 15 Gew.-% erfolgt eine irreversible Schrumpfung der vorher lockeren Struktur des Hydrogels zur dichten Struktur des sog. Xerogels.

Eine zweite, bevorzugt geeignete Gruppe von Kieselsäure-Poliermitteln sind die Fällungskieselsäuren. Diese werden durch Ausfällung von Kieselsäure aus verdünnten Alkalisilikat-Lösungen durch Zugabe von starken Säuren unter Bedingungen erhalten, bei welchen die Aggregation zum Sol und Gel nicht eintreten kann. Geeignete Verfahren zur Bevorzugt geeignet ist eine Fällungskieselsäure mit einer BET-Oberfläche von 15 - 110 m²/g, einer Partikelgröße von 0,5 - 20 µm, wobei wenigstens 80 Gew.-% der Primärpartikel unter 5 µm liegen sollen, und einer Viskosität in 30 %iger Glycerin-Wasser-(1 : 1)-Dispersion von 30 - 60 Pa.s (20°C) in einer Menge von 10 - 20 Gew.-% der Zahnpaste. Bevorzugt geeignete Fällungskieselsäuren dieser Art weisen außerdem gerundete Ecken und Kanten auf und sind unter der Handelsbezeichnung Sident®12 DS (DEGUSSA) erhältlich.

Andere Fällungskieselsäuren dieser Art sind Sident® 8 (DEGUSSA) und Sorbosil® AC 39 (Crosfield Chemicals). Diese Kieselsäuren zeichnen sich durch eine geringere Verdickungswirkung und eine etwas höhere mittlere Teilchengröße von 8 --14 µm bei einer spezifischen Oberfläche von 40 - 75 m²/g (nach BET) aus und eignen sich besonders gut für flüssige Zahncremes. Diese sollten eine Viskosität (25°C, Scherrate D = 10 s⁻¹) von 10 - 100 Pa.s aufweisen.

Zahnpasten, die eine deutlich höhere Viskosität von mehr als 100 Pa.s (25° C, D = 10 s⁻¹) aufweisen, benötigen hingegen einen genügend hohen Anteil an Kieselsäuren mit einer Teilchengröße von weniger als 5 µm, bevorzugt wenigstens 3 Gew.-% einer Kieselsäure mit einer Partikelgröße von 1 - 3 µm. Solchen Zahnpasten setzt man daher bevorzugt neben den genannten Fällungskieselsäuren noch feinteiligere, sogenannte Verdickungskieselsäuren mit einer BET-Oberfläche von 150 --250 m²/g zu, z.B. die Handelsprodukte Sipernat 22 LS oder Sipernat® 320 DS.

Als weitere Poliermittelkomponente kann auch z.B. Aluminiumoxid in Form von schwach calcinierter Tonerde mit einem Gehalt an - und -Aluminiumoxid in einer Menge von ca. 1 - 5 Gew.-% enthalten sein. Ein solches geeignetes Aluminiumoxid ist unter der Handelsbezeichnung "Poliertonerde P10 feinst" (Giulini Chemie) erhältlich.

Als Poliermittel eignen sich weiter alle für Zahnpasten bekannten Reibkörper wie z. B. Natriumaluminiumsilikate wie z. B. Zeolith A, organische Polymere wie z. B. Polymethacrylat oder Gemische dieser und der vorstehend genannten Reibkörper.

Zusammenfassend sind erfindungsgemäße Mittel bevorzugt, die zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthalten.

Die angegebenen Mengen beziehen sich auf die Gesamtmenge an Reibkörpern, wobei einzelne Reibkörper vorzugsweise in engeren Mengenbereichen eingesetzt werden. Erfindungsgemäß bevorzugte Mittel enthalten beispielsweise 5 bis 20 Gew.-%, vorzugsweise 8 bis 21 Gew.-%, weiter bevorzugt 9 bis 20 Gew.-% und insbesondere 11 bis 19 Gew.-% Kieselsäure(n). Weiter bevorzugte erfindungsgemäße Mittel sind dadurch gekennzeichnet, daß sie 0,25 bis 2 Gew.-%, vorzugsweise 0,5 bis 1,5 Gew.-% und insbesondere 0,75 bis 1,25 Gew.-% Aluminiumoxid enthalten.

Mund- und Zahnpflege- und reinigungsmittel, insbesondere Zahnpasten, können z.B. auch Substanzen enthalten, die gegen Plaque und/oder Zahnstein wirksam sind.

Zahnbelag (Plaque) ist ein rauher, klebriger Belag auf den Zähnen, der aus Speichel, Bakterien und Nahrungsresten besteht. Setzen sich Mineralsalze (z.B. Calcium, Phosphat) aus dem Speichel im Zahnbelag ab, so bilden sich harte, weiße oder gelbliche Ablagerungen am Zahn, die man Zahnstein nennt. In dem porösen Zahnstein kann sich wiederum leicht Zahnbelag absetzen, der das Zahnfleisch angreift.

Die Bakterien auf der Zahnoberfläche bauen Kohlenhydrate, besonders Zucker, aus der Nahrung zu Säure ab. Diese Säure löst die Zahnsubstanz auf und es kommt zu Karies (Zahnfäule). Dabei werden besonders die Mineralien Calcium und Phosphat aus dem Zahnschmelz herausgelöst. Nach dem Zahnschmelzmantel werden auch innere Schichten des Zahnes angegriffen. Bakterien können in das Zahnmark eindringen und dort zu Entzündungen führen. Meist kommt es dann zu stechenden Zahnschmerzen.

Wie bereits erwähnt, beinhaltet Plaque Bakterien, so daß sich zur Bekämpfung von Plaque antimikrobielle Stoffe eignen. Diese besitzen darüber hinaus eine Wirkung als Konservierungsmittel.

In Mund und Zahnpflege- und -reinigungsmitteln können beispielsweise die auch in Lebensmitteln zugelassenen Konservierungsmittel Sorbinsäure (E 200), Kaliumsorbat (E 202), Calciumsorbat (E 203), Benzoesäure (E 210), Natriumbenzoat (E 211), Kaliumbenzoat (E 212), Calciumbenzoat (E 213), Ethyl-4-hydroxybenzoat (E 214), Ethyl-4-hydroxybenzoat, Natriumsalz (E 215), Propyl-4-hydroxybenzoat (E 216), Propyl-4-hydroxybenzoat, Natriumsalz (E 217), Methyl-4-hydroxybenzoat (E 218), Methyl-4-hydroxybenzoat, Natriumsalz (E 219), Schwefeldioxid (schweflige Säure), (E 220), Natriumsulfit (E 221), Natriumhydrogensulfit (E 222), Natriumdisulfit (E 223), Kaliumdisulfit (E 224), Calciumsulfit (E 226), Calciumhydrogensulfit (E 227), Kaliumhydrogensulfit (E 228), Biphenyl (E 230), Orthophenylphenol (2-Biphenylol), (E 231), Natriumorthophenylphenolat (E 232), Nisin (E 234), Natamycin (E 235), Ameisensäure (E 236), Natriumformiat (E 237), Calciumformiat (E 238), Hexamethylentetramin (E 239), Dimethyldicarbonat (E 242), Kaliumnitrit (E 249), Natriumnitrit (E 250), Natriumnitrat (E 251), Kaliumnitrat (E 252), Essigsäure (E 260), Kaliumacetat (E 261), Natriumacetat (E 262), Calciumacetat (E 263), Milchsäure (E 270), Propionsäure (E 280), Natriumpropionat (E 281), Calciumpropionat (E 282), Kaliumpropionat (E 283), Borsäure (E 284), Natriumtetraborat (E 285), Hydroxybernsteinsäure (Äpfelsäure), (E 296), Fumarsäure (E 297), Lysozym (E 1105),
eingesetzt werden.

Bevorzugte Stoffe sind ausgewählt aus p-Hydroxybenzoesäuremethyl-, -ethyl- oder propylester, Natriumsorbat, Natriumbenzoat, Bromchlorophen, Triclosan, Phenyl-Salicylsäureester, Biguaniden z. B. Chlorhexidin (1,1'-Hexamethylenbis[5-(4-chlorphenyl)-biguanid), Thymol usw..

Erfindungsgemäß bevorzugte Mittel enthalten zusätzlich Antiplaque-Wirkstoffe, vorzugsweise p-Hydroxybenzoesäuremethyl-, -ethyl- oder propylester, Natriumsorbat, Natriumbenzoat, Bromchlorophen, Triclosan, Phenyl- Salicylsäureester, Biguanide z. B. Chlorhexidin, Thymol, vorzugsweise in Mengen von 0,1 bis 5 Gew.%, vorzugsweise von 0,25 bis 2,5 Gew.% und insbesondere von 0,5 bis 1,5 Gew.%, jeweils bezogen auf das gesamte Mittel.

Die angegebenen Mengen beziehen sich auf die Gesamtmenge an Antiplaque-Wirkstoffen, wobei einzelne Wirkstoffe vorzugsweise in engeren Mengenbereichen eingesetzt werden. Erfindungsgemäß bevorzugte Mittel enthalten beispielsweise 0,1 bis 2 Gew.-%, vorzugsweise 0,2 bis 1,75 Gew.-%, weiter bevorzugt 0,3 bis 1,5 Gew.-% und insbesondere 0,4 bis 1,0 Gew.-% Natriumbenzoat. Weiter bevorzugte erfindungsgemäße Mittel sind dadurch gekennzeichnet, daß sie 0,005 bis 0,1 Gew.-%, vorzugsweise 0,01 bis 0,075 Gew.-% und insbesondere 0,02 bis 0,05 Gew.-% Chlorhexidin enthalten. Chlorhexidin wird vorzugsweise gemeinsam mit Alkylpolyglycosiden (APG) eingesetzt, wobei in bevorzugten erfindungsgemäßen Mitteln als Alkylglykoside solche mit 8-18 C-Atomen in der Alkylgruppe und einem mittleren Oligomerisationsgrad des Glycosidrestes von 1-3 in einer Menge von 0,025 bis 2,5 Gew.-% enthalten sind.

Gegen Zahnstein wirksame Stoffe können beispielsweise Chelatbildner sein wie z. B. Ethylendiamintetraessigsäure und deren Natriumsalze, Pyrophosphat- Salze wie die wasserlöslichen Dialkali- oder Tetraalkalimetallpyrophosphat- Salze, z. B. Na₄P₂O₇, K₄P₂O₇, Na₂K₂P₂O₇, Na₂H₂P₂O₇ und K ₂H₂P₂O₇ oder Polyphosphat-Salze, die z. B. aus wasserlöslichen Alkalimethalltripolyphosphaten wie Natriumtripolyphosphat und Kaliumtripolyphosphat ausgewählt sein können.

Erfindungsgemäß bevorzugte Mittel sind dadurch gekennzeichnet, daß sie zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthalten.

Mund und Zahnpflege- und -reinigungsmittel können darüber hinaus mit besonderem Vorzug Antikaries-Wirkstoffe enthalten. Diese können beispielsweise aus organischen oder anorganischen Fluoriden ausgewählt sein, z. B. aus Natriumfluorid, Kaliumfluorid, Natriummonofluorphosphat und Natriumfluorosilikat. Auch Zinkfluorid, Zinn-(II)-fluorid sind bevorzugt. Bevorzugt sollte eine Menge von 0,01 - 0,2 Gew.-% Fluor in Form der genannten Verbindungen enthalten sein.

Erfindungsgemäße Mittel, die zusätzlich Antikaries-Wirkstoffe, vorzugsweise Fluorverbindung(en), insbesondere Natriumfluorid, Kaliumfluorid, Natriummonofluorphosphat, Zinkfluorid, Zinnfluorid und Natriumfluorosilikat, vorzugsweise in Mengen von 0,01 bis 5 Gew.%, vorzugsweise von 0,1 bis 2,5 Gew.% und insbesondere von 0,2 bis 1,1 Gew.%, jeweils bezogen auf das gesamte Mittel, enthalten, sind erfindungsgemäß bevorzugt.

Als Konsistenzregler (bzw. Bindemittel) dienen z. B. natürliche und/oder synthetische wasserlösliche Polymere wie Alginate, Carragheenate, Traganth, Stärke und Stärkeether, Celluloseether wie z. B. Carboxymethylcellulose (Na-Salz), Hydroxyethylcellulose, Methylhydroxypropylcellulose, Guar, Akaziengum, Agar-Agar, Xanthan- Gum, Succinoglycan-Gum, Johannisbrotmehl, Pectine, wasserlösliche Carboxyvinylpolymere (z. B. Carbopol®-Typen), Polyvinylalkohol, Polyvinylpyrrolidon, Polyethylenglycole, insbesondere solche mit Molekulargewichten von 1 500-1 000 000.

Weitere Stoffe, die sich zur Viskositätskontrolle eignen, sind z. B. Schichtsilikate wie z. B. Montmorillonit-Tone, kolloidale Verdickungskieselsäuren wie z. B. Aerogel-Kieselsäuren, pyrogene Kieselsäuren oder feinstvermahlene Fällungskieselsäuren. Es können auch viskositätsstabilisierende Zusätze aus der Gruppe der kationischen, zwitterionischen oder ampholytischen stickstoffhaltigen Tenside, der hydroxypropylsubstituierten Hydrocolloide oder der Polyethylenglycol/Polypropylenglycol- Copolymere mit einem mittleren Molgewicht von 1000 bis 5000 oder eine Kombination der genannten Verbindungen in den Zahnpasten verwendet werden.

Auch oberflächenaktive Substanzen sind in den Zahnpasten zur Unterstützung der Reinigungswirkung und gewünschtenfalls auch zur Entwicklung von Schaum beim Zähnebürsten sowie zur Stabilisierung der Polierkörperdispersion im Träger in einer Menge von 0,1-5 Gew.-% enthalten.

Geeignete Tenside sind z. B. lineare Natriumalkylsulfate mit 12-18 C-Atomen in der Alkylgruppe. Diese Stoffe weisen zusätzlich eine enzymhemmende Wirkung auf den bakteriellen Stoffwechsel des Zahnbelags auf. Weitere geeignete Tenside sind Alkalisalze, bevorzugt Natriumsalze von Alkylpolyglycolethersulfat mit 12-16 C-Atomen in der linearen Alkylgruppe und 2-6 Glycolethergruppen im Molekül, von linearem Alkan(C₁₂-C₁₈)-sulfonat, von Sulfobernsteinsäuremonoalkyl(C₁₂-C₁₈)-estern, von sulfatisierten Fettsäuremonoglyceriden, sulfatisierten Fettsäurealkanolamiden, Sulfoessigsäurealkyl(C₁₂-C₁₆)-estern, Acylsarcosinen, Acyltauriden und Acylisothionaten mit jeweils 8-18 C-Atomen in der Acylgruppe. Auch zwitterionische, ampholytische und nichtionische Tenside sind geeignet, z. B. Oxethylate von Fettsäuremono- und -diglyceriden, von Fettsäure-Sorbitanestern und Alkyl(oligo)-Glucoside.

Die Mund- und Zahnpflegemittel, insbesondere die Zahnpasten, können auch die Unempfindlichkeit der Zähne steigernde Substanzen enthalten, beispielsweise Kaliumsalze wie z. B. Kaliumnitrat, Kaliumcitrat, Kaliumchlorid, Kaliumbicarbonat und Kaliumoxalat. Erfindungsgemäß bevorzugte Mittel sind dadurch gekennzeichnet, daß sie die Unempfindlichkeit der Zähne steigernde Substanzen, vorzugsweise Kaliumsalze, besonders bevorzugt Kaliumnitrat und/oder Kaliumcitrat und/oder Kaliumchlorid und/oder Kaliumbicarbonat und/oder Kaliumoxalat, vorzugsweise in Mengen von 0,5 bis 20 Gew.%, besonders bevorzugt von 1,0 bis 15 Gew.%, weiter bevorzugt von 1,5 bis 5 Gew.-% und insbesondere von 1,75 bis 2,5 Gew.%, jeweils bezogen auf das gesamte Mittel, enthalten. Besonders bevorzugte erfindungsgemäße Mund und Zahnpflege- und -reinigungsmittel enthalten 0,5 bis 9,5 Gew.-%, vorzugsweise 1 bis 9 Gew.-%, weiter bevorzugt 2 bis 8 Gew.-%, noch weiter bevorzugt 3 bis 7 Gew.-% und insbesondere 4 bis 6 Gew.-% Kaliumsalz(e). Die erfindungsgemäßen Mittel, insbesondere die Zahnpasten, können auch zusätzlich weitere wundheilende und entzündungshemmende Stoffe, z. B. Wirkstoffe gegen Zahnfleischentzündungen, enthalten. Derartige Stoffe können z. B. ausgewählt sein aus Allantoin, Azulen, Kamillenextrakten, Tocopherol, Panthenol, Bisabolol, Salbeiextrakten.

Als nicht-kationische, bakterizide Komponente eignen sich z.B. Phenole, Resorcine, Bisphenole, Salicylanilide und deren halogenierte Derivate, halogenierte Carbanilide und p-Hydroxybenzoesäureester. Besonders bevorzugte antimikrobielle Komponenten sind halogenierte Diphenylether, z.B. 2,4-Dichlor-2'-hydroxydiphenylether, 4,4'-Dichlor-2'-hydroxydiphenylether, 2,4,4'-Tribrom-2'-hydroxydiphenylether und 2,4,4'-Trichlor-2'-hydroxydiphenylether (Triclosan). Sie werden bevorzugt in Mengen von 0,01 - 1 Gew.-% in die erfindungsgemäßen Zahnpflegemittel eingesetzt. Besonders bevorzugt wird Triclosan in einer Menge von 0,01 - 0,3 Gew.-% eingesetzt.

D-Panthenol D - (+) - 2,4-Dihydroxy-N-(3-hydroxypropyl)-3,3-dimethyl-butyramid zeigt eine der Pantothensäure entsprechende biologische Aktivität. Die Pantothensäure (R - (+) - N - (2,4-Dihydroxy-3,3-dimethylbutyryl-ß-alanin) ist eine Vorstufe in der Biosynthese des Coenzyms A und wird zum Vitamin-B-Komplex (B3) gezählt. Die erfindungsgemäßen Zahnpflegemittel enthalten bevorzugt 0,05 - 5 Gew.-% Panthenol oder ein Salz der Pantothensäure.

Retinol (3,7-Dimethyl-9-(2,6,6-trimethyl-1-cyclohexenyl)-2,4,6,8-nonatetraen-1-ol ist der internationale Freiname für Vitamin A1. Anstelle des Retinols kann auch eines seiner Derivate mit ähnlicher biologischer Wirkung, z.B. ein Ester oder die Retinoesäure (Tretinoin), eines ihrer Salze oder ihre Ester verwendet werden. Bevorzugt wird ein Retinol-Ester, insbesondere ein Fettsäureester einer Fettsäure mit 12 - 22 C-Atomen verwendet. Besonders bevorzugt ist Retinol-Palmitat geeignet. Bei Verwendung eines Retinol-Esters, z.B. Retinol-Palmitat mit einer Aktivität von 1,7 10⁶ I.E. pro g ist eine Menge von 0,001 bis 0,1 Gew.-% bevorzugt. Bei Verwendung anderer Retinol-Derivate empfiehlt sich eine Einsatzmenge, die einer Konzentration von 10³ bis 10⁶ I.E. (Internationale Einheiten) pro 100 g entspricht.

Bevorzugte Zahnpflegemittel gemäß der vorliegenden Erfindung enthalten neben Poliermitteln, Fluorverbindungen, Feuchthaltemitteln und Bindemitteln bevorzugt
0,01 - 1 Gew.-% eines halogenierten Diphenylethers
0,05 - 5 Gew.-% Panthenol oder ein Salz der Pantothensäure und
0,01 - 0,1 Gew.-% eines Retinol-Esters, bevorzugt Retinol-Palmitat.

Die erfindungsgemäßen Mittel, insbesondere die Zahnpasten, können auch Substanzen zur Erhöhung des mineralisierenden Potentials enthalten, beispielsweise calciumhaltige Substanzen wie z. B. Calciumchlorid, Calciumacetat und Dicalciumphosphat-Dihydrat. Die Konzentration der calciumhaltigen Substanz hängt von der Löslichkeit der Substanz und dem Zusammenwirken mit anderen in dem Mund- und Zahnpflegemittel enthaltenen Substanzen ab.

Neben den genannten obligatorischen Komponenten können die erfindungsgemäßen Zahnpflegemittel weitere, an sich bekannte Hilfs- und Zusatzstoffe enthalten. Dabei ist ein Zusatzstoff, der als Zahnpastenkomponente seit langem bekannt ist, in den erfindungsgemäßen Zahnpflegemitteln besonders wirksam: Calcium-glycerophosphat, das Calcium-Salz der Glycerin-1-phosphorsäure oder der Glycerin-2-phosphorsäure oder der zur Glycerin-1-phosphorsäure enantiomeren Glycerin-3-phosphorsäure - oder eines Gemisches dieser Säuren. Die Verbindung hat in Zahnpflegemitteln eine remineralisierende Wirkung, da sie sowohl Calcium- als auch Phosphationen liefert. In den erfindungsgemäßen Zahnpflegemitteln wird Calciumglycerophosphat bevorzugt in Mengen von 0,01 - 1 Gew.-% eingesetzt. Insgesamt können die erfindungsgemäßen Zahnreinigungsmittel übliche Hilfsmittel und Zusatzstoffe in Mengen bis zu 10 Gew.-% enthalten.

Die erfindungsgemäßen Zahnpflegemittel können z.B. durch Zusatz von Aromaölen und Süßungsmitteln in ihren organoleptischen Eigenschaften verbessert werden.

Als Aromaöle können alle die für Mund- und Zahnpflegemittel üblichen natürlichen und synthetischen Aromen eingesetzt werden. Natürliche Aromen können sowohl in Form der aus Drogen isolierten natürlichen ätherischen Öle als auch der daraus isolierten Einzelkomponenten enthalten sein.

Geeignete Aromen sind z.B. Pfefferminzöl, Krauseminzöl, Eukalyptusöl, Anisöl, Fenchelöl, Kümmelöl, Menthylacetat, Zimtaldehyd, Anethol, Vanillin, Thymol sowie Mischungen dieser Komponenten.

Geeignete Süßungsmittel sind z.B. Saccharin-Natrium, Natrium-Cyclamat, Sucrose, Lactose, Meltose, Fructose.

Weitere übliche Hilfs- und Zusatzstoffe für Zahnpasten sind
- Oberflächenaktive Stoffe, bevorzugt anionische, zwitterionische, amphotere, nichtionische Tenside oder eine Kombination mehrerer verschiedener Tenside
- Lösungsmittel und Lösungsvermittler, z.B. niedere einwertige oder mehrwertige Alkohole oder Ether, z.B. Ethanol, 1,2-Propylenglycol, Diethylenglycol oder Butyldiglycol
- Pigmente, wie z.B. Titandioxid
- Farbstoffe
- Puffersubstanzen, z.B. primäre, sekundäre oder tertiäre Alkaliphosphate oder Citronen- säure-/Na-Citrat
- weitere wundheilende oder entzündungshemmende Stoffe, z.B. Allantoin, Harnstoff, Azulen, Kamillewirkstoffe, Acetylsalicylsäurederivate oder Rhodanid
- weitere Vitamine wie z.B. Ascorbinsäure, Biotin, Tocopherol oder Rutin
- Mineralsalze wie z.B. Mangan-, Zink- oder Magnesiumsalze.

Eine weitere wichtige Gruppe von Inhaltstoffen, die in den erfindungsgemäßen Mitteln enthalten sein kann, sind die sogenannten bioaktiven Gläser.

Der Begriff "bioaktive Gläser" umfaßt im Rahmen der vorliegenden Anmeldung Gläser, welche biologisch wirksam und/oder biologisch aktiv sind. Die biologische Wirksamkeit eines Glases kann sich beispielsweise in dessen antimikrobiellen Eigenschaften zeigen, biologisch aktives Glas unterscheidet sich von herkömmlichen Kalk-Natrium-Silicat-Gläsern dadurch, daß es lebendes Gewebe bindet. Biologisch aktives Glas bezeichnet dabei beispielsweise ein Glas, das eine feste Bindung mit Körpergewebe eingeht, wobei eine Hydroxyl-Apatitschicht ausgebildet wird. Unter bioaktivem Glas wird auch ein Glas verstanden, das antimikrobielle und/oder entzündungshemmende Wirkung zeigt. Die Glaspulver zeigen gegenüber Bakterien, Pilzen sowie Viren eine biozide bzw. eine biostatische Wirkung; sind im Kontakt mit dem Menschen hautverträglich, toxikologisch unbedenklich und insbesondere auch zum Verzehr geeignet.

Erfindungsgemäß besonders bevorzugte Mittel sind dadurch gekennzeichnet, daß sie 0,2 bis 20 Gew.-%, vorzugsweise 0,4 bis 14 Gew.-%, besonders bevorzugt 0,5 bis 3 Gew.-% und insbesondere 0,6 bis 2 Gew.-% mindestens eines bioaktiven Glases enthalten.

Zur Verstärkung der antimikrobiellen, insbesondere der antibakteriellen Eigenschaften der Glaskeramik können antimikrobiell wirkende Ionen wie z. B. Ag, Au, I, Ce, Cu, Zn in Konzentrationen kleiner 5 Gew.-% enthalten sein.

Farbgebende Ionen wie z. B. Mn, Cu, Fe, Cr, Co, V, können einzeln oder kombiniert, vorzugsweise in einer Gesamtkonzentration kleiner 1 Gew.-%, enthalten sein.

Üblicherweise wird das Glas bzw. die Glaskeramik in Pulverform eingesetzt. Die Keramisierung kann entweder mit einem Glasblock bzw. Glasribbons erfolgen oder aber mit Glaspulver. Nach der Keramisierung müssen die Glaskeramikblöcke oder Ribbons zu Pulver gemahlen werden. Wurde das Pulver keramisiert, muß gegebenenfalls auch erneut gemahlen werden, um Agglomerate, die während des Keramisierungsschrittes entständen sind, zu entfernen. Die Mahlungen können sowohl trocken als auch in wässrigen oder nicht wässrigen Mahlmedien durchgeführt werden. Üblicherweise liegen die Partikelgrößen kleiner 500 µm. Als zweckmäßig haben sich Partikelgrößen < 100 µm bzw. < 20 µm erwiesen. Besonders geeignet sind Partikelgrößen < 10 µm sowie kleiner 5 µm sowie kleiner 2 µm, siehe weiter unten.

Die in den bevorzugten erfindungsgemäßen Zusammensetzungen enthaltenen bioaktiven Gläser bzw. Glaspulver oder Glaskeramikpulver oder Komposit-Zusammensetzungen umfassen Gläser, die bevorzugt nachfolgende Komponenten umfassen: SiO₂: 35-80 Gew.-%, Na₂O: 0-35 Gew.- %, P₂O₅: 0-80 Gew.-%, MgO: 0-5 Gew.-%, Ag₂O: 0-0,5 Gew.-%, AgJ: 0-0,5 Gew.- %, NaJ: 0-5 Gew.-%, TiO₂: 0-5 Gew.-%, K₂O: 0-35 Gew.-%, ZnO: 0-10 Gew.-%, Al₂O₃: 0-25 Gew.-% und B₂O₃: 0-25 Gew.-% .

Weiterhin können dem Grundglas gemäß obiger Zusammensetzung zur Erzielung weiterer Effekte wie beispielsweise Farbigkeit oder UV-Filterung Ionen wie Fe, Co, Cr, V, Ce, Cu, Mn, Ni, Bi, Sn, Ag, Au, J einzeln oder in Summe bis zu 10 Gew.-% zugegeben werden. Eine weiter Glaszusammensetzung kann wie folgt sein: SiO₂: 35-80 Gew.-%, Na₂O: 0-35 Gew.-%, P₂O₅: 0-80 Gew.-%, MgO: 0-5 Gew.- %, Ag₂O: 0-0,5 Gew.-%, AgJ: 0-0,5 Gew.-%, NaJ: 0-5 Gew.-%, TiO₂: 0-5 Gew.-%, K₂O: 0-35 Gew.-%, ZnO: 0-10 Gew.-%, Al₂ O₃: 0-25 Gew.-%, B₂O₃: 0-25 Gew.-%, SnO: 0-5 Gew.-%, CeO₂: 0-3 Gew.- % und Au: 0,001-0,1 Gew.-%. Besonders bevorzugte erfindungsgemäße Mund- und Zahnpflege- und -reinigungsmittel sind dadurch gekennzeichnet, daß das bioaktive Glas - bezogen auf sein Gewicht - folgende Zusammensetzung aufweist:

| | | | |
|---|---|---|---|
| SiO₂ | 35 bis 60 Gew.-%, | vorzugsweise | 40 bis 60 Gew.-%, |
| Na₂O | 0 bis 35 Gew.-%, | vorzugsweise | 5 bis 30 Gew.-%, |
| K₂O | 0 bis 35 Gew.-%, | vorzugsweise | 0 bis 20 Gew.-%, |
| P₂O₅ | 0 bis 10 Gew.-%, | vorzugsweise | 2 bis 10 Gew.-%, |
| MgO | 0 bis 10 Gew.-%, | vorzugsweise | 0 bis 5 Gew.-%, |
| CaO | 0 bis 35 Gew.-%, | vorzugsweise | 5 bis 30 Gew.-%, |
| Al₂O₃ | 0 bis 25 Gew.-%, | vorzugsweise | 0 bis 5 Gew.-%, |
| B₂O₃ | 0 bis 25 Gew.-%, | vorzugsweise | 0 bis 5 Gew.-%, |
| TiO₂ | 0 bis 10 Gew.-%, | vorzugsweise | 0,1 bis 5 Gew.-%. |

Wie bereits weiter oben erwähnt, wird das bioaktive Glas vorzugsweise in partikulärer Form eingesetzt. Hier sind besonders bevorzugte erfindungsgemäße Mund- und Zahnpflege- und - reinigungsmittel dadurch gekennzeichnet, daß das antimikrobielle Glas Teilchengrößen < 10 µm, vorzugsweise von 0,5 bis 4 µm, besonders bevorzugt von 1 bis 2 µm, aufweist.

## Patentansprüche

1. Mund- und Zahnpflege- und -reinigungsmittel, enthaltend einen fließfähigen Träger und darin suspendierte Partikel, **dadurch gekennzeichnet, dass** die im fließfähigen Träger suspendierten Partikel
- mindestens anteilsweise aus wasserunlöslichen synthetischen Polymeren bestehen und
- stäbchenförmig sind, d.h. in einer Raumrichtung eine Ausdehnung aufweisen, die mindestens doppelt, vorzugsweise mindestens dreifach, besonders bevorzugt mindestens vierfach und insbesondere mindestens fünffach so groß ist wie die größere der Ausdehnungen in die beiden anderen Raumrichtungen
- in der größten räumlichen Ausdehnung Längen zwischen 100 und 10.000 µm aufweisen.

2. Mund- und Zahnpflege- und -reinigungsmittel, enthaltend einen fließfähigen Träger und darin suspendierte Partikel, **dadurch gekennzeichnet, dass** die im fließfähigen Träger suspendierten Partikel
- mindestens anteilsweise aus wasserunlöslichen synthetischen Polymeren bestehen und
- plättchenförmig sind, d.h. in zwei Raumrichtungen eine Ausdehnung aufweisen, die mindestens doppelt, vorzugsweise mindestens dreifach, besonders bevorzugt mindestens vierfach und insbesondere mindestens fünffach so groß ist wie die Ausdehnung in die andere Raumrichtung
- in der größten räumlichen Ausdehnung Längen zwischen 100 und 10.000 µm aufweisen.

3. Mund- und Zahnpflege- und -reinigungsmittel nach einem der Ansprüche 1 oder 2, **dadurch gekennzeichnet, dass** die im fließfähigen Träger suspendierten Partikel mindestens anteilsweise aus Polyester und/oder mindestens anteilsweise aus Polyvinylchlorid und/oder mindestens anteilsweise aus Polyethylenterphthalat und/oder mindestens anteilsweise aus Polyurethan und/oder aus einem Blend aus Polyethylenterephthalat(en) und Polyacrylat(en) und/oder aus einem Blend aus Polyethylenterephthalat(en) und Polyurethan(en) bestehen.

4. Mund- und Zahnpflege- und -reinigungsmittel nach einem der Ansprüche 1 bis 3, **dadurch gekennzeichnet, dass** die im fließfähigen Träger suspendierten Partikel mindestens anteilsweise aus Kompositmaterialien aus synthetischen Polymeren und Metall(en) bestehen.

5. Mund- und Zahnpflege- und -reinigungsmittel nach einem der Ansprüche 1 bis 4, **dadurch gekennzeichnet, dass** der fließfähige Träger - bezogen auf sein Gewicht - 0,1 bis 75 Gew.-%, vorzugsweise 1 bis 70 Gew.-%, besonders bevorzugt 10 bis 65 Gew.-%, weiter bevorzugt 15 bis 60 Gew.-% und insbesondere 20 bis 60 Gew.-% Wasser enthält.

6. Mund- und Zahnpflege- und -reinigungsmittel nach einem der Ansprüche 1 bis 5, **dadurch gekennzeichnet, dass** die im fließfähigen Träger suspendierten Partikel - bezogen auf ihr Gewicht - 0,01 bis 20 Gew.-% Duftstoff(e) und/oder Aromen enthalten.

7. Mund- und Zahnpflege- und -reinigungsmittel nach einem der Ansprüche 1 bis 6, **dadurch gekennzeichnet, dass** die im fließfähigen Träger suspendierten Partikel - bezogen auf ihr Gewicht - 0,05 bis 19 Gew.-%, vorzugsweise 0,1 bis 18 Gew.-%, besonders bevorzugt 0,5 bis 17 Gew.-%, weiter bevorzugt 0,75 bis 16 Gew.-% und insbesondere 1 bis 15 Gew.-% mindestens eines Aromas aus der Gruppe Pfefferminz, Spearmint, Menthol, Limone, Limette, Apfel, Orange, Zitrone, Erdbeere, Aloe Vera, Kaugummi enthalten.

8. Mund- und Zahnpflege- und -reinigungsmittel nach einem der Ansprüche 1 bis 7, **dadurch gekennzeichnet, dass** die im fließfähigen Träger suspendierten Partikel - bezogen auf ihr Gewicht - zusätzlich 0,01 bis 5 Gew.-%, vorzugsweise 0,05 bis 2,5 Gew.-% und insbesondere 0,1 bis 1 Gew.-% Tensid(e) enthalten, wobei bevorzugte Tenside ausgewählt sind aus Fettalkoholsulfaten, insbesondere Natriumlaurylsulfat, und/oder Betainen, insbesondere Cocoamidopropylbetain.

9. Mund- und Zahnpflege- und -reinigungsmittel nach einem der Ansprüche 1 bis 8, **dadurch gekennzeichnet, dass** die im fließfähigen Träger suspendierten Partikel in der größten räumlichen Ausdehnung Längen zwischen 200 und 8000 µm, besonders bevorzugt zwischen 500 und 7000 µm und insbesondere zwischen 1000 und 5000 µm aufweisen.

10. Mund und Zahnpflege- und -reinigungsmittel nach einem der Ansprüche 1 bis 9, **dadurch gekennzeichnet, dass** es 0,5 bis 9,5 Gew.-%, vorzugsweise 1 bis 9 Gew.-%, weiter bevorzugt 2 bis 8 Gew.-%, noch weiter bevorzugt 3 bis 7 Gew.-% und insbesondere 4 bis 6 Gew.-% Kaliumsalz(e) enthält.

11. Mund und Zahnpflege- und -reinigungsmittel nach einem der Ansprüche 1 bis 10, **dadurch gekennzeichnet, dass** es - bezogen auf sein Gewicht - 10 bis 60 Gew.-%, vorzugsweise 12,5 bis 50 Gew.-%, besonders bevorzugt 15 bis 45 Gew.-% und insbesondere 20 bis 40 Gew.-% mindestens eines mehrwertigen Alkohols aus der Gruppe Sorbit und/oder Glycerin und/oder 1,2-Propylenglycol.-% enthält.

12. Mund- und Zahnpflege- und -reinigungsmittel gemäß einem der Ansprüche 1 bis 11, **dadurch gekennzeichnet, dass** es zusätzlich Putzkörper, vorzugsweise Kieselsäuren, Aluminiumhydroxid, Aluminiumoxid, Calciumpyrophosphat, Kreide, Dicalciumphosphat-dihydrat (CaHPO₄ ·2H₂O), Natriumaluminiumsilikate, insbesondere Zeolith A, organische Polymere, insbesondere Polymethacrylate oder Gemische dieser Reibkörper, vorzugsweise in Mengen von 1 bis 30 Gew.%, vorzugsweise von 2,5 bis 25 Gew.% und insbesondere von 5 bis 22 Gew.%, jeweils bezogen auf das gesamte Mittel, enthält.

13. Mund- und Zahnpflege- und -reinigungsmittel nach einem der Ansprüche 1 bis 12, **dadurch gekennzeichnet, dass** es zusätzlich Phosphat(e), vorzugsweise Alkalimetallphosphat(e) und insbesondere Natriumtripolyphosphat, vorzugsweise in Mengen von 1 bis 10 Gew.-%, besonders bevorzugt von 2 bis 8 Gew.-% und insbesondere von 3 bis 7 Gew.-%, jeweils bezogen auf das gesamte Mittel, enthält.

## Claims

1. An oral and dental care and cleaning agent, containing a flowable carrier and particles suspended therein, **characterized in that** the particles suspended in the flowable carrier
- consist at least in part of water-insoluble synthetic polymers and
- are rod-shaped, i.e. have an expansion in one spatial direction that is at least two times, preferably at least three times, particularly preferably at least four times, and in particular at least five times, larger than the largest expansion in the other two spatial directions
- have lengths between 100 and 10,000 µm in the largest spatial expansion.

2. An oral and dental care and cleaning agent, containing a flowable carrier and particles suspended therein, **characterized in that** the particles suspended in the flowable carrier
- consist at least in part of water-insoluble synthetic polymers and
- are plate-like, i.e. have an expansion in two spatial directions that is at least two times, preferably at least three times, particularly preferably at least four times, and in particular at least five times, larger than the largest expansion in the other spatial direction
- have lengths between 100 and 10,000 µm in the largest spatial expansion.

3. The oral and dental care and cleaning agent according to one of claims 1 or 2, **characterized in that** the particles suspended in the flowable carrier consist at least in part of polyester and/or at least in part of polyvinyl chloride and/or at least in part of polyethylene terephthalate and/or at least in part of polyurethane and/or of a blend of polyethylene terephthalate(s) and polyacrylate(s) and/or of a blend of polyethylene terephthalate(s) and polyurethane(s).

4. The oral and dental care and cleaning agent according to one of claims 1 to 3, **characterized in that** the particles suspended in the flowable carrier consist at least in part of composite materials made of synthetic polymers and metal(s).

5. The oral and dental care and cleaning agent according to one of claims 1 to 4, **characterized in that** the flowable carrier contains, based on its weight, 0.1 to 75 wt.%, preferably 1 to 70 wt.%, particularly preferably 10 to 65 wt.%, more preferably 15 to 60 wt.%, and in particular 20 to 60 wt.%, water.

6. The oral and dental care and cleaning agent according to one of claims 1 to 5, **characterized in that** the particles suspended in the flowable carrier contain, based on their weight, 0.01 to 20 wt.% fragrance(s) and/or flavors.

7. The oral and dental care and cleaning agent according to one of claims 1 to 6, **characterized in that** the particles suspended in the flowable carrier contain, based on their weight, 0.05 to 19 wt.%, preferably 0.1 to 18 wt.%, particularly preferably 0.5 to 17 wt.%, more preferably 0.75 to 16 wt.%, and in particular 1 to 15 wt.%, at least one flavor from the group of peppermint, spearmint, menthol, citrus, lime, apple, orange, lemon, strawberry, aloe vera or chewing gum.

8. The oral and dental care and cleaning agent according to one of claims 1 to 7, **characterized in that** the particles suspended in the carrier also contain, based on their weight, 0.01 to 5 wt.%, preferably 0.05 to 2.5 wt.%, and in particular 0.1 to 1 wt.%, surfactant(s), preferred surfactants being selected from fatty alcohol sulfates, in particular sodium lauryl sulfate, and/or betaines, in particular cocamidopropyl betaine.

9. The oral and dental care and cleaning agent according to one of claims 1 to 8, **characterized in that** the particles suspended in the flowable carrier have lengths between 200 and 8000 µm, particularly preferably between 500 and 7000 µm, and in particular between 1000 and 5000 µm, in the largest spatial expansion.

10. The oral and dental care and cleaning agent according to one of claims 1 to 9, **characterized in that** it contains 0.5 to 9.5 wt.%, preferably 1 to 9 wt.%, more preferably 2 to 8 wt.%, even more preferably 3 to 7 wt.%, and in particular 4 to 6 wt.%, potassium salt(s).

11. The oral and dental care and cleaning agent according to one of claims 1 to 10, **characterized in that** it contains, based on its weight, 10 to 60 wt.%, preferably 12.5 to 50 wt.%, particularly preferably 15 to 45 wt.% and in particular 20 to 40 wt.%, of at least one polyvalent alcohol from the group of sorbitol and/or glycerol and/or 1,2-propylene glycol-%.

12. The oral and dental care and cleaning agent according to one of claims 1 to 11, **characterized in that** it also comprises cleaning substances, preferably silicic acids, aluminum hydroxide, aluminum oxide, calcium pyrophosphate, chalk, dicalcium phosphate dihydrate (CaHPO₄·2H₂O), sodium aluminosilicates, in particular zeolite A, organic polymers, in particular polymethacrylates, or mixtures of these abrasives, preferably in amounts of 1 to 30 wt.%, preferably 2.5 to 25 wt.%, and in particular 5 to 22 wt.%, in each case based on the total agent.

13. The oral and dental care and cleaning agent according to one of claims 1 to 12, **characterized in that** it also comprises phosphate(s), preferably alkali metal phosphate(s), and in particular sodium tripolyphosphate, preferably in amounts of 1 to 10 wt.%, particularly preferably 2 to 8 wt.%, and in particular 3 to 7 wt.%, in each case based on the total agent.

## Revendications

1. Agent de nettoyage et de soins buccodentaires contenant un support fluide et des particules en suspension dans ce dernier, **caractérisé en ce que** les particules en suspension dans le support fluide
- comprennent au moins en partie des polymères synthétiques insolubles dans l'eau et
- se présentent sous la forme de tiges, c'est-à-dire qu'elles présentent dans une direction spatiale une dimension qui est au moins deux fois, de préférence au moins trois fois, plus préférablement au moins quatre fois et en particulier au moins cinq fois, plus grande que la plus grande des dimensions dans les deux autres directions spatiales,
- et ont, dans la plus grande dimension spatiale, des longueurs comprises entre 100 et 10 000 µm).

2. Agent de nettoyage et de soins buccodentaires contenant un support fluide et des particules en suspension dans ce dernier, **caractérisé en ce que** les particules en suspension dans le support fluide
- comprennent au moins en partie des polymères synthétiques insolubles dans l'eau et
- se présentent sous la forme de plaquettes, c'est-à-dire qu'elles présentent dans deux direction spatiale une dimension qui est au moins deux fois, de préférence au moins trois fois, plus préférablement au moins quatre fois et en particulier au moins cinq fois, plus grande que la dimension dans l'autre direction spatiale,
- et ont, dans la plus grande dimension spatiale, des longueurs comprises entre 100 et 10 000 µm).

3. Agent de nettoyage et de soins buccodentaires selon l'une des revendications 1 ou 2, **caractérisé en ce que** les particules en suspension dans le support fluide comprennent au moins en partie du polyester et/ou au moins en partie du polychlorure de vinyle et/ou au moins en partie du polyéthylène téréphtalate et/ou au moins en partie du polyuréthane et/ou un mélange de polyacrylate(s) et de polyéthylène téréphtalate(s) et/ou un mélange de polyuréthane(s) et de polyéthylène téréphtalate(s) .

4. Agent de nettoyage et de soins buccodentaires selon l'une des revendications 1 à 3, **caractérisé en ce que** les particules en suspension dans le support fluide comprennent au moins en partie des matières composites formées de polymères synthétiques et d'au moins un métal.

5. Agent de nettoyage et de soins buccodentaires selon l'une des revendications 1 à 4, **caractérisé en ce que** le support fluide contient, sur la base de son poids, de 0,1 à 75 % en poids, de préférence de 1 à 70 % en poids, de manière particulièrement préférée de 10 à 65 % en poids, plus préférablement de 15 à 60 % en poids et en particulier de 20 à 60 % en poids d'eau.

6. Agent de nettoyage et de soins buccodentaires selon l'une des revendications 1 à 5, **caractérisé en ce que** les particules en suspension dans le support fluide contiennent, sur la base de leur poids, de 0,01 à 20 % en poids d'au moins un parfum et/ou d'arômes.

7. Agent de nettoyage et de soins buccodentaires selon l'une des revendications 1 à 6, **caractérisé en ce que** les particules en suspension dans le support fluide contiennent, sur la base de leur poids, de 0,05 à 19 % en poids, de préférence 0,1 à 18 % en poids, de manière particulièrement préférée de 0,5 à 17 % en poids, plus préférablement de 0,75 à 16 % en poids et en particulier de 1 à 15 % en poids d'au moins un arôme du groupe comprenant la menthe poivrée, la menthe verte, le menthol, le citron vert, la limette, la pomme, l'orange, le citron, la fraise, l'aloe vera, le chewing-gum.

8. Agent de nettoyage et de soins buccodentaires selon l'une des revendications 1 à 7, **caractérisé en ce que** les particules en suspension dans le support fluide contiennent en outre, sur la base de leur poids, de 0,01 à 5 % en poids, de préférence de 0,05 à 2,5 % en poids et en particulier de 0,1 à 1 % en poids d'au moins un tensioactif, les tensioactifs préférés étant choisis parmi les sulfates d'alcools gras, en particulier le lauryl sulfate de sodium, et/ou les bétaïnes, en particulier la cocoamidopropylbétaïne.

9. Agent de nettoyage et de soins buccodentaires selon l'une des revendications 1 à 8, **caractérisé en ce que** les particules en suspension dans le support fluide ont, dans la plus grande dimension, des longueurs allant de 200 à 8000 µm), plus préférablement de 500 à 7000 µm et en particulier entre 1000 et 5000 µm).

10. Agent de nettoyage et de soins buccodentaires selon l'une des revendications 1 à 9, **caractérisé en ce qu'**il contient de 0,5 à 9,5 % en poids, de préférence de 1 à 9 % en poids, plus préférablement de 2 à 8 % en poids, encore plus préférablement de 3 à 7 % en poids et en particulier de 4 à 6 % en poids d'au moins un sel de potassium.

11. Agent de nettoyage et de soins buccodentaires selon l'une des revendications 1 à 10, **caractérisé en ce qu'**il contient, sur la base de son poids, de 10 à 60 % en poids, de préférence de 12,5 à 50 % en poids, plus préférablement de 15 à 45 % en poids et en particulier de 20 à 40 % en poids d'au moins un alcool polyvalent du groupe comprenant le sorbitol et/ou le glycérol et/ou le 1,2-propylène-glycol.

12. Agent de nettoyage et de soins buccodentaires selon l'une des revendications 1 à 11, **caractérisé en ce qu'**il contient en outre des abrasifs, de préférence des acides siliciques, de l'hydroxyde d'aluminium, de l'alumine, du pyrophosphate de calcium, de la craie, du phosphate dicalcique dihydraté (CaHPO₄·2H₂O), des silicates d'aluminium et de sodium, en particulier de la zéolite A, des polymères organiques, en particulier des polyméthacrylates ou des mélanges de ces éléments abrasifs, de préférence dans des quantités allant de 1 à 30 % en poids, de préférence de 2,5 à 25 % et en particulier de 5 à 22 % en poids, à chaque fois sur la base de tout l'agent.

13. Agent de nettoyage et de soins buccodentaires selon l'une des revendications 1 à 12, **caractérisé en ce qu'**il contient en outre au moins un phosphate, de préférence au moins un phosphate de métal alcalin et en particulier du tripolyphosphate de sodium, de préférence dans des quantités allant de 1 à 10% en poids, de manière particulièrement préférée de 2 à 8 % en poids et en particulier de 3 à 7 % en poids, à chaque fois sur la base de tout l'agent.
